(19) Europäisches Patentamt
European Patent Office
Office européen des brevets

(11) EP 4 349 417 A1

(12) **EUROPEAN PATENT APPLICATION**
published in accordance with Art. 153(4) EPC

(43) Date of publication:
**10.04.2024 Bulletin 2024/15**

(21) Application number: **22816098.2**

(22) Date of filing: **31.05.2022**

(51) International Patent Classification (IPC):
***A61Q 17/04*** (2006.01) ***A61K 8/11*** (2006.01)
***A61K 8/25*** (2006.01) ***A61K 8/34*** (2006.01)
***A61K 8/37*** (2006.01) ***A61K 8/73*** (2006.01)
***A61K 8/81*** (2006.01) ***A61K 8/898*** (2006.01)

(52) Cooperative Patent Classification (CPC):
**A61K 8/02; A61K 8/11; A61K 8/25; A61K 8/34; A61K 8/37; A61K 8/49; A61K 8/73; A61K 8/81; A61K 8/85; A61K 8/898; A61Q 17/04; A61Q 19/00**

(86) International application number:
**PCT/JP2022/022097**

(87) International publication number:
**WO 2022/255352 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2021 JP 2021091624**
**18.01.2022 JP 2022005970**

(71) Applicant: **Kao Corporation**
**Chuo-ku**
**Tokyo 103-8210 (JP)**

(72) Inventors:
- **KURIHARA, Jun**
**Tokyo 131-8501 (JP)**
- **HIRONO, Shingo**
**Tokyo 131-8501 (JP)**
- **ITO, Norihiro**
**Wakayama-shi, Wakayama 640-8580 (JP)**
- **HIRATA, Tomohiro**
**Wakayama-shi, Wakayama 640-8580 (JP)**
- **TAKAZAWA, Nobuo**
**Tokyo 131-8501 (JP)**

(74) Representative: **Hoffmann Eitle**
**Patent- und Rechtsanwälte PartmbB**
**Arabellastraße 30**
**81925 München (DE)**

# (54) DERMATOLOGICAL TOPICAL AGENT

(57) Provided is a dermatological topical agent which has excellent use impression, such as fresh feeling at the beginning of application, and which can form a good coating film of a non-volatile oil agent, such as an oil-soluble UV absorber.

The dermatological topical agent comprises granular gel capsules comprising a non-volatile oil agent, and a liquid comprising the following component (bl): (b1) a volatile medium, wherein the granular gel capsules are dispersed in the liquid, a content of the non-volatile oil agent in the granular gel capsules is 1.5 mass% or more based on a total mass of the dermatological topical agent, a content of the non-volatile oil agent in the liquid is 0 mass% or more and 14 mass% or less based on the total mass of the dermatological topical agent, and a content of an aqueous thickener in the liquid is 0 mass% or more and 0.25 mass% or less based on the total mass of the dermatological topical agent.

EP 4 349 417 A1

## Description

Field of the Invention

The present invention relates to a dermatological topical agent.

Background of the Invention

Conventionally, dermatological topical agents in which granular gel capsules with a UV absorber dispersed therein are dispersed in a liquid have been known as dermatological topical agents having UV protection efficacy. As such a dermatological topical agent, for example, a skin lotion prepared by mixing 1.00 mass% of polyoxyethylene hardened castor oil, 0.20 mass% of an (acrylic acid/C10-30 alkyl acrylate) copolymer, and 0.10 mass% of xanthan gum, together with PVA microcapsules containing a UV absorber and water has been reported (Patent Literature 1).

(Patent Literature 1) JP-A-2002-37713
(Patent Literature 2) JP-A-3-193716
(Patent Literature 3) JP-A-2012-171891
(Patent Literature 4) JP-A-2012-171892
(Patent Literature 5) JP-A-2014-91737
(Patent Literature 6) JP-A-2016-104712

Summary of the Invention

The present invention provides a dermatological topical agent comprising granular gel capsules comprising a non-volatile oil agent, and a liquid comprising the following component (b1):
(b1) a volatile medium,

wherein the granular gel capsules are dispersed in the liquid,
a content of the non-volatile oil agent in the granular gel capsules is 1.5 mass% or more based on a total mass of the dermatological topical agent,
a content of the non-volatile oil agent in the liquid is 0 mass% or more and 14 mass% or less based on the total mass of the dermatological topical agent, and
a content of an aqueous thickener in the liquid is 0 mass% or more and 0.25 mass% or less based on the total mass of the dermatological topical agent.

Brief Description of the Drawings

Fig. 1 is a micrograph of a model plate not coated with a dermatological topical agent.
Fig. 2 is a fluorescence micrograph of a model plate coated with an agent $\alpha$.

Detailed Description of the Invention

The skin lotion disclosed in Patent Literature 1 had a heavy texture at the beginning of application, and it was difficult to give fresh feeling.

Other known products are a two-layered suntan cosmetic containing a microcapsule layer and a water layer, obtained by dispersing a UV absorber and the like in an aqueous gelatine solution (Patent Literature 2), and dermatological topical agents in which granular gel capsules with a UV absorber dispersed therein are dispersed in an oil-in-water type emulsion composition highly mixed with an oil-soluble UV absorber and the like (Patent Literatures 3 to 6).

However, the two-layered suntan cosmetic disclosed in Patent Literature 2 had insufficient UV protection efficacy. The dermatological topical agents disclosed in Patent Literatures 3 to 6 were prone to give uneven distribution of UV absorbers during application, resulting in insufficient UV protection efficacy in some cases.

The present invention relates to providing a dermatological topical agent which has excellent use impression, such as fresh feeling at the beginning of application, and which can form a good coating film of a non-volatile oil agent, such as an oil-soluble UV absorber.

The present inventor found that a dermatological topical agent containing granular gel capsules containing a

non-volatile oil agent, and a liquid containing a volatile medium, wherein the granular gel capsules are dispersed in the liquid, a content of the non-volatile oil agent in the granular gel capsules is 1.5 mass% or more based on a total mass of the dermatological topical agent, a content of the non-volatile oil agent in the liquid is 0 mass% or more and 14 mass% or less based on the total mass of the dermatological topical agent, and a content of an aqueous thickener in the liquid is 0 mass% or more and 0.25 mass% or less based on the total mass of the dermatological topical agent, has excellent use impression, such as fresh feeling at the beginning of application, and can from a good coating film of a non-volatile oil agent, such as an oil-soluble UV absorber. Thus, the present invention has been completed.

The dermatological topical agent of the present invention has excellent use impression, such as fresh feeling at the beginning of application, and can from a good coating film of a non-volatile oil agent, such as an oil-soluble UV absorber.

The dermatological topical agent of the present invention is characterized by containing granular gel capsules containing a non-volatile oil agent (hereinafter also referred to as the component (A)), and a liquid containing the following component (b1): (b1) a volatile medium (hereinafter also referred to as the component (B)), wherein the granular gel capsules are dispersed in the liquid, a content of the non-volatile oil agent in the granular gel capsules is 1.5 mass% or more based on a total mass of the dermatological topical agent, a content of the non-volatile oil agent in the liquid is 0 mass% or more and 14 mass% or less based on the total mass of the dermatological topical agent, and a content of an aqueous thickener in the liquid is 0 mass% or more and 0.25 mass% or less based on the total mass of the dermatological topical agent.

In the present specification, oily components are referred to as "oil agents", and the oil agents may be solid, semi-solid, or liquid. For example, oil-soluble UV absorbers, amphiphilic solid fats, and oily thickeners are oil agents. The "non-volatile oil agent" mentioned above refers to an oily component which is not volatile at 25°C under 1 atm. For example, non-volatile oil-soluble UV absorbers, non-volatile amphiphilic solid fats, and non-volatile oily thickeners are non-volatile oil agents.

In the present specification, being non-volatile at 25°C under 1 atm is referred to as being "non-volatile", and being volatile at 25°C under 1 atm is referred to as being "volatile". Specifically, "non-volatile" means that the evaporation percentage is less than 20% at 25°C for 6 hours, as measured by the following method.

Measurement method: A 90-mm-diameter filter paper is placed in a 120-mm-diameter glass petri dish, and 1 g of a sample is placed on the filter paper and stored in room with a RH of 65% (25°C) for 6 hours. The mass of the sample before and after storage is measured, and the evaporation percentage is calculated by the following equation:

$$\text{Evaporation percentage (\%)} = [(\text{sample mass before storage}) - (\text{sample mass after storage})]/(\text{sample mass before storage}) \times 100$$

"Volatile" means that the evaporation percentage is more than 20% at 25°C for 6 hours, as measured by the method described above.

The content of the non-volatile oil agent in the granular gel capsules is 1.5 mass% or more based on the total mass of the dermatological topical agent of the present invention. By thus setting the content of the non-volatile oil agent to 1.5 mass% or more, the uniformity of the coating film can be improved.

The content of the non-volatile oil agent in the granular gel capsules is preferably 3 mass% or more, more preferably 6 mass% or more, further more preferably 9 mass% or more, and particularly preferably 12 mass% or more, based on the total mass of the dermatological topical agent of the present invention, in terms of the uniformity of the coating film and the like. Further, in terms of use impression and the like, the content of the non-volatile oil agent is preferably 40 mass% or less, more preferably 35 mass% or less, further more preferably 30 mass% or less, and particularly preferably 25 mass% or less, based on the total mass of the dermatological topical agent of the present invention. The specific range is preferably 3 mass% or more and 40 mass% or less, more preferably 6 mass% or more and 35 mass% or less, further more preferably 9 mass% or more and 30 mass% or less, and particularly preferably 12 mass% or more and 25 mass% or less, based on the total mass of the dermatological topical agent of the present invention.

<(A) Granular Gel Capsules>

The granular gel capsules are preferably granular hydrogel capsules. In the present specification, "granular hydrogel capsules" refer to granular gel capsules in which an oil phase is dispersed in a hydrogel. "Hydrogel" refers to a gel obtained from an encapsulating agent using water as a solvent. The concept of "granular hydrogel capsules" does not encompass capsules containing an outer skin as an outer layer and a core component as an inner layer, wherein

the inner and outer layers are concentric.

The granular hydrogel capsules are preferably those in which an oil phase containing a non-volatile oil agent is dispersed in a hydrogel, more preferably those in which an oil phase containing an oil-soluble UV absorber is dispersed in a hydrogel, and particularly preferably those containing a continuous phase of a non-crosslinked hydrogel and an oil phase dispersed in the continuous phase, the oil phase containing an oil-soluble UV absorber. The oil phase is preferably one containing an amphiphilic solid fat and an oily thickener, together with an oil-soluble UV absorber.

In the present specification, "non-crosslinked hydrogel" refers to one which undergoes gelation due to the thermal reversibility of sol-gel. The non-crosslinked hydrogel preferably has a dissolution temperature in water of 75°C or more, and more preferably 75°C or more and 90°C or less. It is also preferable that the gelling temperature be 30°C or more and 45°C or less when cooled after being dissolved in water.

In terms of storage stability (stabilization of the granular gel capsules), fresh feeling at the beginning of application, good adhesive feeling in the later stages of application, and the like, the granular gel capsules are preferably those containing one or more selected from the group consisting of (a1) a UV absorber (preferably an oil-soluble UV absorber), (a2) an encapsulating agent, (a3) water, (a4) an amphiphilic solid fat, (a5) a thickener (examples of the thickener (a5) include (a5-1) an oily thickener and (a5-2) an aqueous thickener), (a6) polyhydric alcohol, (a7) a powder, and (a8) a non-volatile oil agent (excluding oil-soluble UV absorbers, amphiphilic solid fats, and oily thickeners); more preferably those containing (a1) a UV absorber (preferably an oil-soluble UV absorber) and further containing, in addition to the UV absorber (a1) (preferably an oil-soluble UV absorber), one or more selected from the group consisting of (a2) an encapsulating agent, (a3) water, (a4) an amphiphilic solid fat, (a5) a thickener, (a6) polyhydric alcohol, (a7) a powder, and (a8) a non-volatile oil agent (excluding oil-soluble UV absorbers, amphiphilic solid fats, and oily thickeners); further more preferably those containing the components (a1) and (a2); further more preferably those containing the components (a1) to (a3); further more preferably those containing the components (a1) to (a4); further more preferably those containing the components (a1) to (a5); further more preferably those containing the components (a1) to (a5) and one or more selected from the group consisting of the components (a6) and (a7); further more preferably those containing the components (a1) to (a6); and particularly preferably those containing the components (a1) to (a7).

When the granular gel capsules contain an encapsulating agent, the granular gel capsules are preferably those in which the component (a1) is dispersed in a gel-like encapsulating agent, more preferably those in which the components (a1) and (a3) are dispersed in a gel-like encapsulating agent, further more preferably those in which the components (a1), (a3), and (a4) are dispersed in a gel-like encapsulating agent, further more preferably those in which the component (a1) and the components (a3) to (a5) are dispersed in a gel-like encapsulating agent, further more preferably those in which the component (a1), the components (a3) to (a5), and one or more selected from the group consisting of the components (a6) and (a7) are dispersed in a gel-like encapsulating agent, further more preferably those in which the component (a1) and the components (a3) to (a6) are dispersed in a gel-like encapsulating agent, and particularly preferably those in which the component (a1) and the components (a3) to (a7) are dispersed in a gel-like encapsulating agent.

Further, in terms of use impression and UV protection efficacy, the granular gel capsules are preferably those containing, as the non-volatile oil agent, one or more selected from the group consisting of (a1) an oil-soluble UV absorber, (a4) an amphiphilic solid fat, (a5-1) an oily thickener, and (a8) a non-volatile oil agent (excluding oil-soluble UV absorbers, amphiphilic solid fats, and oily thickeners); more preferably those containing at least (a1) an oil-soluble UV absorber as the non-volatile oil agent; further more preferably those containing, as the non-volatile oil agents, (a1) an oil-soluble UV absorber and one or more selected from the group consisting of (a4) an amphiphilic solid fat and (a5-1) an oily thickener; and particularly preferably those containing (a1) an oil-soluble UV absorber, (a4) an amphiphilic solid fat, and (a5-1) an oily thickener as the non-volatile oil agents.

((a1) UV Absorber)

The UV absorber is preferably an oil-soluble UV absorber. Because the granular gel capsules contain a UV absorber, stickiness at the beginning of application derived from the UV absorber can be suppressed, and a uniform coating film is formed in the process of collapse of the granular gel capsules, thereby improving UV protection efficacy.

The UV absorber is preferably an organic UV absorber, in terms of water resistance, abrasion resistance, and the like.

Examples of organic UV absorbers include benzoate UV absorbers, anthranilate UV absorbers, salicylate UV absorbers, cinnamate UV absorbers, benzophenone UV absorbers, triazine UV absorbers, silicone UV absorbers, and the like. Among these, one or more selected from the group consisting of benzoate UV absorbers, cinnamate UV absorbers, and triazine UV absorbers are preferred, in terms of UV protection efficacy, photostability, and the like.

Examples of benzoate UV absorbers include para-aminobenzoic acid (PABA), glyceryl PABA, ethyl dihydroxypropyl PABA, N-ethoxylate PABA ethyl ester, N-dimethyl PABA ethyl ester, N-dimethyl PABA butyl ester, N-dimethyl PABA amyl ester, octyl dimethyl PABA, hexyl diethylaminohydroxybenzoylbenzoate, and the like.

Examples of anthranilate UV absorbers include homomenthyl-N-acetylanthranilate and the like.

Examples of salicylate UV absorbers include amyl salicylate, menthyl salicylate, homomenthyl salicylate, octyl salicylate, phenyl salicylate, benzyl salicylate, p-isopropanol phenyl salicylate, and the like.

Examples of cinnamate UV absorbers include octyl cinnamate, ethyl-4-isopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate, 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, glyceryl mono-2-ethylhexanoyl diparamethoxycinnamate, and the like.

Examples of benzophenone UV absorbers include 2,4-dihydroxybenzophenone, 2,2'-dihydroxy-4-methoxybenzophenone, 2,2'-dihydroxy-4,4'-dihydroxybenzophenone, 2-hydroxy-4-methoxybenzophenone, 2-hydroxy-4-methoxy-4'-methylbenzophenone, 2-hydroxy-4-methoxybenzophenone, 4-phenylbenzophenone, 2-ethylhexyl 4'-phenylbenzophenone-2-carboxylate, 2-hydroxy-4-n-octoxybenzophenone, 4-hydroxy-3-carboxybenzophenone, and the like.

Examples of triazine UV absorbers include 2,4,6-tris[4-(2-ethylhexyloxycarbonyl)anilino]-1,3,5-triazine, dioctyl butamido triazone, 2,4-bis-[{4-(2-ethylhexyloxy)-2-hydroxy}-phenyl]-6-(4-methoxyphenyl)-1,3,5-triazine, and the like.

Examples of silicone UV absorbers include dimethicodiethylbenzalmalonate and the like.

Examples of other organic UV absorbers include 3-(4'-methylbenzylidene)-dl-camphor, 3-benzylidene-dl-camphor, urocanic acid ethyl ester, 2-phenyl-5-methylbenzoxazole, 2,2'-hydroxy-5-methylphenylbenzotriazole, 2-(2'-hydroxy-5-t-octylphenyl)benzotriazole, dibenzalazine, dianisoylmethane, 4-methoxy-4'-t-butyldibenzoylmethane, 5-(3,3-dimethyl-2-norbonylidene)-3-pentan-2-one, the benzene/bis-1,3-diketone derivative described in JP-A-2-212579, the benzoyl pinacolone derivative described in JP-A-1991-220153, and the like.

Organic UV absorbers can be broadly classified into organic UV absorbers which are solid at 25°C under 1 atm, and organic UV absorbers which are liquid at 25°C under 1 atm. For example, hexyl diethylaminohydroxybenzoylbenzoate, 2,4-bis-[{4-(2-ethylhexyloxy)-2-hydroxy}-phenyl]-6-(4-methoxyphenyl)-1,3,5-triazine, 2,4,6-tris[4-(2-ethylhexyloxycarbonyl)anilino]-1,3,5-triazine, and the like mentioned above are organic UV absorbers which are solid at 25°C under 1 atm. Isopropyl-p-methoxycinnamate, 2-ethylhexyl-p-methoxycinnamate, 2-ethoxyethyl-p-methoxycinnamate, and the like mentioned above are organic UV absorbers which are liquid at 25°C under 1 atm.

The UV absorbers may be used singly or in combinations of two or more thereof.

The content of the UV absorber (preferably an oil-soluble UV absorber) in the granular gel capsules is preferably 1.5 mass% or more, more preferably 3 mass% or more, further more preferably 6 mass% or more, further more preferably 9 mass% or more, and particularly preferably 12 mass% or more, based on the total mass of the dermatological topical agent of the present invention, in terms of UV protection efficacy and the like. Further, in terms of use impression and the like, the content of the UV absorber is preferably 40 mass% or less, more preferably 35 mass% or less, further more preferably 30 mass% or less, and particularly preferably 25 mass% or less, based on the total mass of the dermatological topical agent of the present invention. The specific range is preferably 1.5 mass% or more and 40 mass% or less, more preferably 3 mass% or more and 40 mass% or less, further more preferably 6 mass% or more and 35 mass% or less, further more preferably 9 mass% or more and 30 mass% or less, and particularly preferably 12 mass% or more and 25 mass% or less, based on the total mass of the dermatological topical agent of the present invention.

With a content of the UV absorber in the granular gel capsules of 12 mass% or more, UV protection efficacy is particularly improved.

The content ratio of the UV absorber (preferably an oil-soluble UV absorber) in the granular gel capsules is preferably 15 mass% or more, more preferably 17 mass% or more, further more preferably 19 mass% or more, and particularly preferably 22 mass% or more, based on the total mass of the granular gel capsules, in terms of UV protection efficacy and the like. Further, in terms of use impression, emulsion stability, and the like, the content ratio of the UV absorber is preferably 54 mass% or less, more preferably 48 mass% or less, further more preferably 42 mass% or less, and particularly preferably 40 mass% or less, based on the total mass of the granular gel capsules. The specific range is preferably 15 mass% or more and 54 mass% or less, more preferably 17 mass% or more and 48 mass% or less, further more preferably 19 mass% or more and 42 mass% or less, and particularly preferably 22 mass% or more and 40 mass% or less, based on the total mass of the granular gel capsules. By thus increasing the content ratio of the UV absorber in the granular gel capsules, all of fresh feeling at the beginning of application, watery feeling at the beginning of application, and UV protection efficacy can be further improved. In addition, sticky feeling and friction feeling can be suppressed.

The content mass ratio of the UV absorber (preferably an oil-soluble UV absorber) in the component (a1) granular gel capsules to the total of all UV absorbers in the dermatological topical agent of the present invention, [(a1)/(all UV absorbers)], is preferably 0.6 or more and 1 or less, more preferably 0.85 or more and 1 or less, further more preferably 0.9 or more and 1 or less, further more preferably 0.95 or more and 1 or less, further more preferably 0.99 or more and 1 or less, and particularly preferably 1, in terms of fresh feeling at the beginning of application, watery feeling at the beginning of application, UV protection efficacy, and the like. By thus increasing the content mass ratio [(a1)/(all UV absorbers)], all of fresh feeling at the beginning of application, watery feeling at the beginning of application, and UV protection efficacy can be further improved. In addition, sticky feeling and friction feeling can be suppressed.

The "total of all UV absorbers in the dermatological topical agent" means the total of UV absorbers inside the granular gel capsules and UV absorbers outside the granular gel capsules.

((a2) Encapsulating Agent)

The encapsulating agent preferably contains a water-soluble non-crosslinked polymer. Examples of such water-soluble non-crosslinked polymers include agar, carrageenan, gelatin, gellan gum, xanthan gum, high-methoxyl pectin, and the like. Among these, the encapsulating agent is preferably one or more selected from the group consisting of agar, carrageenan, and gelatin, and particularly preferably agar, in terms of good use impression of watery feeling and coolness, storage stability (stabilization of the granular gel capsules), and the like. The "agar" in the present application refers to hemicellulose containing galactan composed of 1,3 and 1,4 bonds of galactose.

The jelly strength of agar (Nikkansui method) is preferably 147 kPa (1 500 g/$cm^2$) or less, and more preferably 19.6 kPa (200 g/$cm^2$) or more and 127 kPa (1 300 g/$cm^2$) or less, in terms of use impression and the like.

The jelly strength can be determined in the following manner. A 1.5 mass% aqueous solution of agar is prepared, and the aqueous solution is left at 20°C for 15 hours to solidify a gel, which is then cut into a size of 25 mm $\times$ 25 mm $\times$ 3 mm (length $\times$ width $\times$ height). A load is applied to the cut gel using a Nikkansui type jelly strength tester (manufactured by Kiya-Seisakusho Co., Ltd.), and the jelly strength can be determined as the maximum mass (g) per $cm^2$ of the surface area when the gel withstands the load at 20°C for 20 seconds.

Examples of commercial products of agar include INA KANTEN PS-84, Z-10, AX-30, AX-100, AX-200, T-1, S-5, M-7, UP-16, CS-16A, CS-420, CS-670, and XY-908 (all of which are manufactured by Ina Food Industry Co., Ltd.) and the like.

Carrageenan is a linear galactan composed of alternately repeating $\alpha$ (1->3) and $\beta$ (1->4) bonds. The $\beta$ (1->4)-linked galactose units are present in part or in whole as 3,6-anhydro-D-galactose and its sulfate ester. There are $\kappa$-carrageenan, ι-carrageenan, and $\lambda$-carrageenan depending on the composition structure, and any of these can be used.

Gelatin is a denatured protein obtained by solubilizing collagen (a main component of animal connective tissues) with an acid or an alkali, followed by heat-denaturation.

The encapsulating agents may be used singly or in combinations of two or more thereof.

The content of the encapsulating agent in the granular gel capsules is preferably 0.05 mass% or more, more preferably 0.1 mass% or more, further more preferably 0.15 mass% or more, and particularly preferably 0.2 mass% or more, based on the total mass of the dermatological topical agent of the present invention, in terms of storage stability, watery feeling at the beginning of application, and the like. Further, in terms of the uniformity of the coating film, UV protection efficacy, appearance during application, ease of collapse, and the like, the content of the encapsulating agent is preferably 2 mass% or less, more preferably 1.75 mass% or less, further more preferably 1.5 mass% or less, and particularly preferably 1 mass% or less, based on the total mass of the dermatological topical agent of the present invention. The specific range is preferably 0.05 mass% or more and 2 mass% or less, more preferably 0.1 mass% or more and 1.75 mass% or less, further more preferably 0.15 mass% or more and 1.5 mass% or less, and particularly preferably 0.2 mass% or more and 1 mass% or less, based on the total mass of the dermatological topical agent of the present invention.

With a content of the encapsulating agent in the granular gel capsules of 1 mass% or less, the uniformity of the coating film and UV protection efficacy are particularly improved.

The content ratio of the encapsulating agent in the granular gel capsules is preferably 0.05 mass% or more, more preferably 0.1 mass% or more, further more preferably 0.2 mass% or more, and particularly preferably 0.4 mass% or more, based on the total mass of the granular gel capsules, in terms of storage stability and the like. Further, in terms of the uniformity of the coating film, UV protection efficacy, redispersibility (difficulty in caking), and the like, the content ratio of the encapsulating agent is preferably 10 mass% or less, more preferably 7.5 mass% or less, further more preferably 5 mass% or less, further more preferably 3 mass% or less, and particularly preferably 2.5 mass% or less, based on the total mass of the granular gel capsules. The specific range is preferably 0.05 mass% or more and 10 mass% or less, more preferably 0.1 mass% or more and 7.5 mass% or less, further more preferably 0.2 mass% or more and 5 mass% or less, further more preferably 0.4 mass% or more and 3 mass% or less, and particularly preferably 0.4 mass% or more and 2.5 mass% or less, based on the total mass of the granular gel capsules.

With a content ratio of the encapsulating agent in the granular gel capsules of 2.5 mass% or less, redispersibility (difficulty in caking) is particularly improved. Even if the dermatological topical agent has low viscosity at 25°C, good redispersibility (difficulty in caking) is achieved. In addition, the uniformity of the coating film and UV protection efficacy are also improved.

In the granular gel capsules, the content mass ratio of the component (a2) encapsulating agent to the component (a1) UV absorber (preferably an oil-soluble UV absorber), [(a2)/(a1)], is preferably 0.001 or more, more preferably 0.003 or more, further more preferably 0.005 or more, and particularly preferably 0.01 or more, in terms of storage stability and the like. Further, in terms of the uniformity of the coating film, UV protection efficacy, use impression (unlikely to

feel rough to touch, soft, and easy to collapse), and the like, the content mass ratio is preferably 0.2 or less, more preferably 0.1 or less, further more preferably 0.08 or less, and particularly preferably 0.06 or less. The specific range is preferably 0.001 or more and 0.2 or less, more preferably 0.003 or more and 0.1 or less, further more preferably 0.005 or more and 0.08 or less, and particularly preferably 0.01 or more and 0.06 or less.

In the granular gel capsules, the content mass ratio of the total of all non-volatile oil agents to the component (a2) encapsulating agent, [(all non-volatile oil agents in granular gel capsules)/(a2)], is preferably 5 or more, more preferably 10 or more, further more preferably 15 or more, and particularly preferably 20 or more, in terms of smoothness during application and the like. Further, in terms of storage stability and the like, the content mass ratio is preferably 105 or less, more preferably 95 or less, further more preferably 85 or less, and particularly preferably 75 or less. The specific range is preferably 5 or more and 105 or less, more preferably 10 or more and 95 or less, further more preferably 15 or more and 85 or less, and particularly preferably 20 or more and 75 or less.

((a3) Water)

The content of water in the granular gel capsules is preferably 5 mass% or more, more preferably 7.5 mass% or more, further more preferably 10 mass% or more, and particularly preferably 15 mass% or more, based on the total mass of the dermatological topical agent of the present invention, in terms of fresh feeling at the beginning of application, watery feeling at the beginning of application, stability (gel stability), and the like. The content of water in the granular gel capsules is also preferably 65 mass% or less, more preferably 60 mass% or less, further more preferably 55 mass% or less, and particularly preferably 50 mass% or less, based on the total mass of the dermatological topical agent of the present invention. The specific range is preferably 5 mass% or more and 65 mass% or less, more preferably 7.5 mass% or more and 60 mass% or less, further more preferably 10 mass% or more and 55 mass% or less, and particularly preferably 15 mass% or more and 50 mass% or less, based on the total mass of the dermatological topical agent of the present invention.

The content ratio of water in the granular gel capsules is preferably 25 mass% or more, more preferably 30 mass% or more, further more preferably 35 mass% or more, and particularly preferably 40 mass% or more, based on the total mass of the granular gel capsules, in terms of fresh feeling at the beginning of application, watery feeling at the beginning of application, stability (gel stability), and the like. The content ratio of water in the granular gel capsules is also preferably 90 mass% or less, more preferably 85 mass% or less, further more preferably 80 mass% or less, and particularly preferably 75 mass% or less, based on the total mass of the granular gel capsules. The specific range is preferably 25 mass% or more and 90 mass% or less, more preferably 30 mass% or more and 85 mass% or less, further more preferably 35 mass% or more and 80 mass% or less, and particularly preferably 40 mass% or more and 75 mass% or less, based on the total mass of the granular gel capsules.

In the granular gel capsules, the content mass ratio of the component (a3) water to the component (a1) UV absorber (preferably an oil-soluble UV absorber) , [(a3)/(a1)], is preferably 0.25 or more, more preferably 0.5 or more, further more preferably 0.75 or more, and particularly preferably 1 or more, in terms of stability (gel stability) and the like. Further, in terms of the uniformity of the coating film, UV protection efficacy, emulsion stability, and the like, the content mass ratio is preferably 5 or less, more preferably 4.5 or less, further more preferably 4 or less, and particularly preferably 3.5 or less. The specific range is preferably 0.25 or more and 5 or less, more preferably 0.5 or more and 4.5 or less, further more preferably 0.75 or more and 4 or less, and particularly preferably 1 or more and 3.5 or less.

((a4) Amphiphilic Solid Fat)

Examples of amphiphilic solid fats include ceramides, alcohols having from 12 to 22 carbon atoms, polyhydric alcohol mono C12-22 fatty acid esters, polyhydric alcohol mono C12-22 alkyl ethers, and the like.

When the granular gel capsules contain an encapsulating agent and an amphiphilic solid fat, the amphiphilic solid fat forms a uniform coating film using the encapsulating agent as a scaffold upon the collapse of the granular gel capsules during application. When the granular gel capsules further contain a UV absorber, particularly good UV protection efficacy can be achieved.

Examples of ceramides include one or more selected from the group consisting of natural ceramides and pseudo-ceramides. Preferred ceramides are those described in JP-A-2013-53146, in terms of storage stability (stabilization of the granular gel capsules) and no friction feeling.

Specific examples of natural ceramides include Ceramide Types 1 to 7 obtained by amidating sphingosine, dihydrosphingosine, phytosphingosine, or sphingadienine (e.g., pig and human ceramides shown in Figure 2 of J. Lipid Res., 24: 759 (1983) and Figure 4 of J. Lipid. Res., 35: 2069 (1994)).

Natural ceramides also include N-alkyl forms (e.g., N-methyl forms) of these ceramides.

These ceramides may be used in the natural (D(-) form) optically active form, in the non-natural (L(+) form) optically active form, or in a mixture of natural and non-natural forms. The relative configuration of the above compounds

may be a natural configuration, a non-natural configuration other than the natural configuration, or a mixture thereof.

Preferred among natural ceramides are compounds of CERAMIDE 1, CERAMIDE 2, CERAMIDE 3, CERAMIDE 5, and CERAMIDE 6II (all of which are listed in INCI, 8th Edition) and compounds of the following formulas:

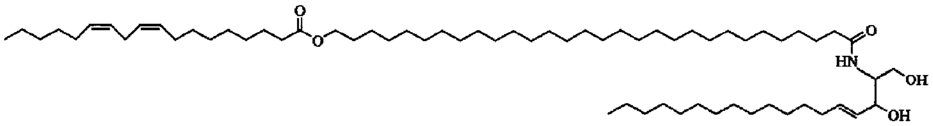

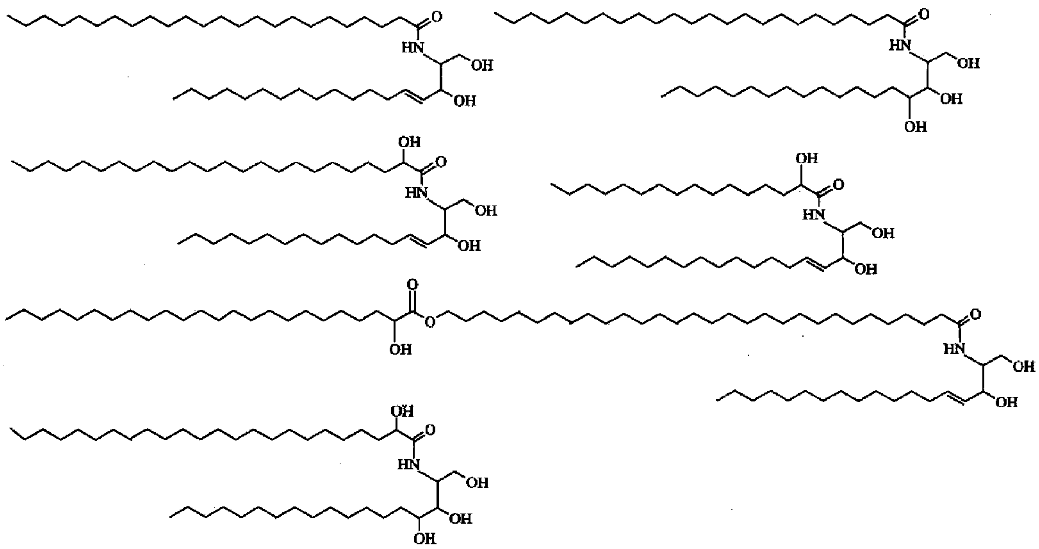

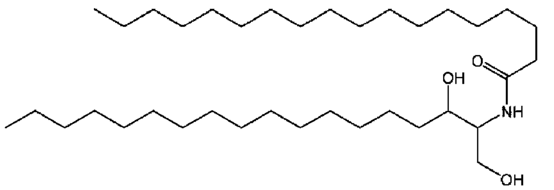

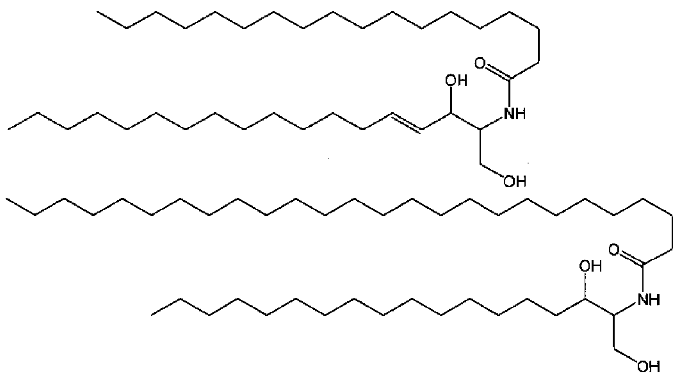

These can be either natural extracts or synthesized products, and commercial products can be used.

Examples of commercial products of such natural ceramides include Ceramide I, Ceramide III, Ceramide IIIA, Ceramide IIIB, Ceramide IIIC, and Ceramide VI (all of which are manufactured by Cosmo Farm), Ceramide TIC-001 (manufactured by Takasago International Corporation), CERAMIDE II (manufactured by Quest International), DS-Ceramide VI, DS-CLA-Phytoceramide, C6-Phytoceramide, and DS-ceramide Y3S (manufactured by Doosan), and CERAMIDE 2 (manufactured by Sederma).

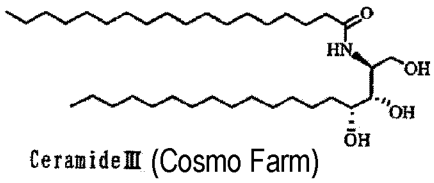

CeramideIII (Cosmo Farm)

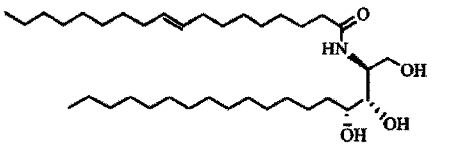

CeramideIIIB (Cosmo Farm)

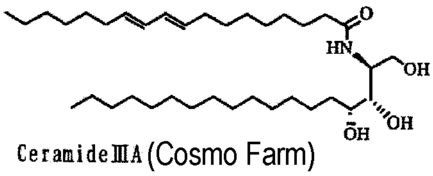

CeramideIIIA (Cosmo Farm)

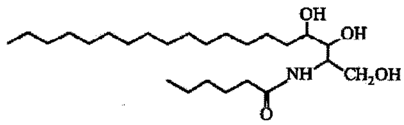

Phytoceramide (Doosan)

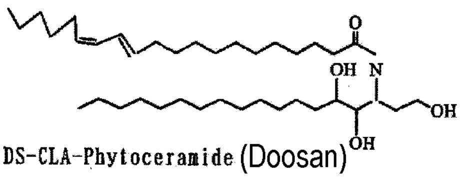

DS-CLA-Phytoceramide (Doosan)

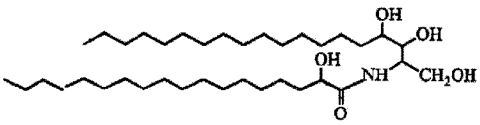

DS-Ceramide VI (Doosan)

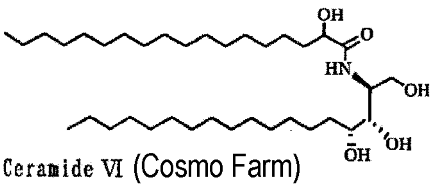

Ceramide VI (Cosmo Farm)

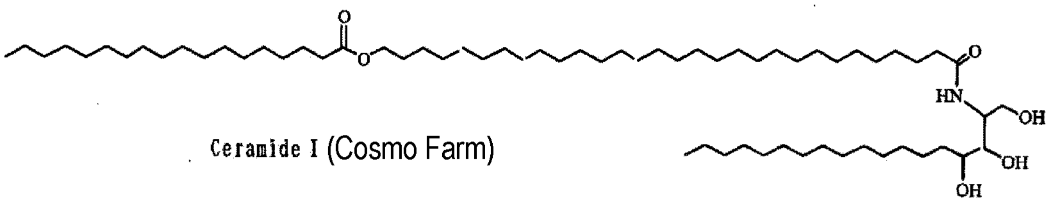

Ceramide I (Cosmo Farm)

The pseudo-ceramide is preferably a pseudo-ceramide of the following formula (1), in terms of storage stability (stabilization of the granular gel capsules) and no friction feeling.

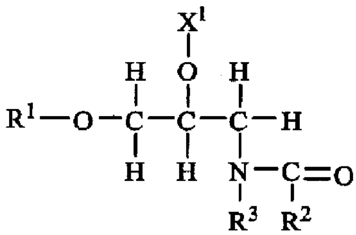

(1)

[In the formula (1),

$R^1$ represents a linear, branched, or cyclic saturated or unsaturated hydrocarbon group having from 10 to 22 carbon atoms and optionally substituted with a hydroxyl group, or a hydrogen atom;
$X^1$ represents a hydrogen atom, an acetyl group, or a glyceryl group;
$R^2$ represents a linear, branched, or cyclic saturated or unsaturated hydrocarbon group (the hydrocarbon group is a preferably an alkyl group) having from 5 to 22 carbon atoms and optionally substituted with a hydroxyl group or an amino group, or a group in which a linear or branched saturated or unsaturated fatty acid having from 8 to 22 carbon atoms and optionally substituted with a hydroxyl group is ester-bonded to the ω-end of the hydrocarbon group (the hydrocarbon group is preferably an alkyl group); and

$R^3$ represents a hydrogen atom or a hydrocarbon group (the hydrocarbon group is preferably an alkyl group) with a total number of carbon atoms of from 1 to 30 optionally substituted with a hydroxyl group, a hydroxyalkoxy group, an alkoxy group, or an acetoxy group.]

Preferred among pseudo-ceramides are one of the formula (1) wherein $R^1$ is a hexadecyl group, $X^1$ is a hydrogen atom, $R^2$ is a pentadecyl group, and $R^3$ is a hydroxyethyl group; one of the formula (1) wherein $R^1$ is a hexadecyl group, $X^1$ is a hydrogen atom, $R^2$ is a nonyl group, and $R^3$ is a hydroxyethyl group; one of the formula (1) wherein $R^1$ is a hexadecyl group, $X^1$ is a glyceryl group, $R^2$ is a tridecyl group, and $R^3$ is a 3-methoxypropyl group; one of the formula (1) wherein $R^1$ is a hydrogen atom, $X^1$ is a hydrogen atom, $R^2$ is a pentadecyl group, and $R^3$ is a dodecyl group; and one of the formula (1) wherein $R^1$ is a hydrogen atom, $X^1$ is a hydrogen atom, $R^2$ is a 1-hydroxyheptyl group, and $R^3$ is a dodecyl group.

More preferred are one of the formula (1) wherein $R^1$ is a hexadecyl group, $X^1$ is a hydrogen atom, $R^2$ is a pentadecyl group, and $R^3$ is a hydroxyethyl group; one of the formula (1) wherein $R^1$ is a hexadecyl group, $X^1$ is a hydrogen atom, $R^2$ is a nonyl group, and $R^3$ is a hydroxyethyl group; and one of the formula (1) wherein $R^1$ is a hexadecyl group, $X^1$ is a glyceryl group, $R^2$ is a tridecyl group, and $R^3$ is a 3-methoxypropyl group.

Particularly preferred is one of the formula (1) wherein $R^1$ is a hexadecyl group, $X^1$ is a hydrogen atom, $R^2$ is a pentadecyl group, and $R^3$ is a hydroxyethyl group (N-(hexadecyloxyhydroxypropyl)-N-hydroxyethylhexadecanamide).

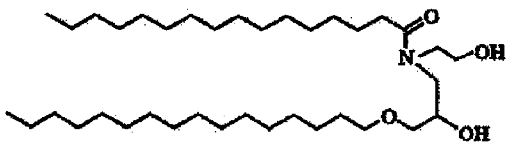

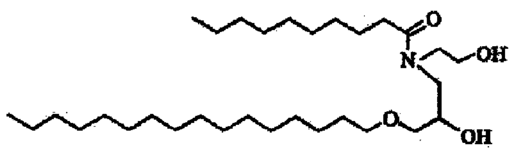

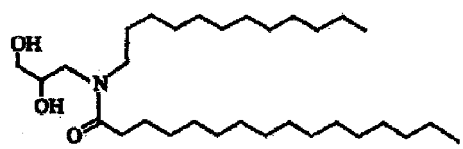

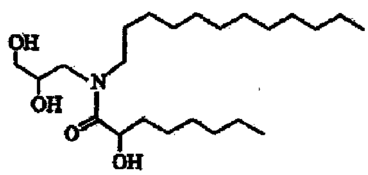

The alcohol having from 12 to 22 carbon atoms is preferably a monovalent alcohol having from 12 to 22 carbon atoms, more preferably a monovalent alcohol having from 14 to 22 carbon atoms, further more preferably a monovalent alcohol having from 16 to 22 carbon atoms, and particularly preferably a monovalent alcohol having from 16 to 18 carbon atoms. The alcohol having from 12 to 22 carbon atoms may be linear or branched, and may be a saturated alcohol or an unsaturated alcohol; however, a linear saturated or unsaturated alcohol is preferred.

Examples of the alcohol having from 12 to 22 carbon atoms include myristyl alcohol, cetyl alcohol, stearyl alcohol, oleyl alcohol, behenyl alcohol, and the like. Among these, preferred are cetyl alcohol and stearyl alcohol.

Examples of the polyhydric alcohol mono C12-22 fatty acid ester include glycerin mono C12-22 fatty acid esters, sorbitan mono C12-22 fatty acid esters, and the like.

The fatty acid residue of the polyhydric alcohol mono C12-22 fatty acid ester is preferably a fatty acid residue having from 14 to 22 carbon atoms, and more preferably a fatty acid residue having from 16 to 22 carbon atoms. The fatty acid residue may be a saturated fatty acid residue or an unsaturated fatty acid residue, and may also be a linear fatty acid residue or a branched fatty acid residue.

Examples of glycerin mono C12-22 fatty acid esters include glycerin monolaurate, glycerin monomyristate, glycerin monopalmitate, glycerin monostearate, glycerin monobehenate, glycerin monooleate, glycerin monoisostearate,

and the like. Among these, preferred is one or more selected from the group consisting of glycerin monopalmitate, glycerin monostearate, and glycerin monobehenate.

Examples of sorbitan mono C12-22 fatty acid esters include sorbitan monolaurate, sorbitan monomyristate, sorbitan monopalmitate, sorbitan monostearate, sorbitan monobehenate, and the like.

Preferred polyhydric alcohol mono C12-22 alkyl ethers are mono C12-22 alkyl glyceryl ethers, more preferred are mono C14-22 alkyl glyceryl ethers, and particularly preferred are mono C16-22 alkyl glyceryl ethers.

Examples of mono C12-22 alkyl glyceryl ethers include monolauryl glyceryl ether, monomyristyl glyceryl ether, monocetyl glyceryl ether, monostearyl glyceryl ether, monobehenyl glyceryl ether, and the like.

Among these components (a4), in terms of storage stability (stabilization of the granular gel capsules) and no friction feeling, one or more selected from the group consisting of monovalent alcohols having from 14 to 22 carbon atoms, glycerin mono C14-22 fatty acid esters, sorbitan mono C14-22 fatty acid esters, and mono C14-22 alkyl glyceryl ethers are preferred; one or more selected from the group consisting of monovalent alcohols having from 16 to 22 carbon atoms, glycerin mono C16-22 fatty acid esters, sorbitan mono C16-22 fatty acid esters, and mono C16-22 alkyl glyceryl ethers are more preferred; one or more selected from the group consisting of monovalent alcohols having from 16 to 22 carbon atoms and glycerin mono C16-22 fatty acid esters are further more preferred; and one or more selected from the group consisting of cetyl alcohol, stearyl alcohol, and lipophilic glycerin monostearate are particularly preferred.

When these are used as the components (a4), the granular gel capsules can have an $\alpha$-gel structure, storage stability can be improved, and friction feeling can be reduced.

The amphiphilic solid fats may be used singly or in combinations of two or more thereof.

The content of the amphiphilic solid fat in the granular gel capsules is preferably 0.1 mass% or more, more preferably 0.3 mass% or more, further more preferably 1 mass% or more, and particularly preferably 1.25 mass% or more, based on the total mass of the dermatological topical agent of the present invention, in terms of the uniformity of the coating film, UV protection efficacy, and storage stability (resistance to leakage of the oil phase). Further, in terms of storage stability and emulsion stability, the content of the amphiphilic solid fat is preferably 12 mass% or less, more preferably 9 mass% or less, further more preferably 6 mass% or less, and particularly preferably 3 mass% or less, based on the total mass of the dermatological topical agent of the present invention. The specific range is preferably 0.1 mass% or more and 12 mass% or less, more preferably 0.3 mass% or more and 9 mass% or less, further more preferably 1 mass% or more and 6 mass% or less, and particularly preferably 1.25 mass% or more and 3 mass% or less, based on the total mass of the dermatological topical agent of the present invention.

The content ratio of the amphiphilic solid fat in the granular gel capsules is preferably 0.1 mass% or more, more preferably 0.5 mass% or more, further more preferably 1 mass% or more, and particularly preferably 2 mass% or more, based on the total mass of the granular gel capsules, in terms of the uniformity of the coating film, UV protection efficacy, storage stability, and the like. Further, in terms of storage stability, emulsion stability, and the like, the content ratio of the amphiphilic solid fat is preferably 12.5 mass% or less, more preferably 10 mass% or less, further more preferably 7.5 mass% or less, and particularly preferably 5 mass% or less, based on the total mass of the granular gel capsules. The specific range is preferably 0.1 mass% or more and 12.5 mass% or less, more preferably 0.5 mass% or more and 10 mass% or less, further more preferably 1 mass% or more and 7.5 mass% or less, and particularly preferably 2 mass% or more and 5 mass% or less, based on the total mass of the granular gel capsules.

With a content ratio of the amphiphilic solid fat in the granular gel capsules of 0.1 mass% or more, the uniformity of the coating film is further improved. When a UV absorber is used in combination, the UV absorber is likely to be uniformly distributed, and UV protection efficacy is particularly improved.

In the granular gel capsules, the content mass ratio of the component (a4) amphiphilic solid fat to the component (a1) UV absorber (preferably an oil-soluble UV absorber), [(a4)/(a1)], is preferably 0.001 or more, more preferably 0.005 or more, further more preferably 0.01 or more, and particularly preferably 0.05 or more, in terms of the uniformity of the coating film, UV protection efficacy, and the like. Further, in terms of emulsion stability, no stickiness during application, and the like, the content mass ratio is preferably 1.2 or less, more preferably 0.9 or less, further more preferably 0.3 or less, and particularly preferably 0.15 or less. The specific range is preferably 0.001 or more and 1.2 or less, more preferably 0.005 or more and 0.9 or less, further more preferably 0.01 or more and 0.3 or less, and particularly preferably 0.05 or more and 0.15 or less.

((a5) Thickener)

Thickeners are broadly classified into (a5-1) an oily thickener and (a5-2) an aqueous thickener.

Examples of the oily thickener include sugar fatty acid ester-based oily thickeners, such as inulin fatty acid esters and dextrin fatty acid esters, as well as polyglyceryl isostearate, glyceryl (behenate/eicosanedioate), organically modified clay minerals, and the like. These may be used singly or in combinations of two or more thereof.

Among these, in terms of the uniformity of the coating film, UV protection efficacy, and the like, sugar fatty acid ester-based oily thickeners are preferred. The fatty acid residue in the sugar fatty acid ester-based oily thickener is

preferably a linear or branched saturated fatty acid residue. The number of carbon atoms in the fatty acid residue is preferably from 8 to 24, more preferably from 12 to 22, and particularly preferably from 14 to 20.

The sugar fatty acid ester-based oily thickener is preferably a dextrin fatty acid ester. Specific examples thereof include dextrin myristate, dextrin palmitate, dextrin stearate, dextrin (palmitate/2-ethylhexanoate), dextrin (palmitate/hexyldecanoate), and the like.

Examples of the aqueous thickener include dextrin, methylcellulose, ethylcellulose, hydroxyethylcellulose, hydroxypropylcellulose, polyvinyl alcohol, polyacrylic acid, polymethacrylic acid, carboxyvinyl polymers, acrylic acid/alkyl acrylate copolymers, xanthan gum, carboxymethyl chitin, chitosan, and the like.

Among these, in terms of emulsion stability during production and the like, one or more selected from the group consisting of polyvinyl alcohol and (meth)acrylic acid-based polymers is preferred; one or more selected from the group consisting of polyvinyl alcohol, carboxyvinyl polymers, and acrylic acid/alkyl acrylate copolymers is particularly preferred; and one or more selected from the group consisting of polyvinyl alcohol and (acrylic acid/C10-30 alkyl acrylate) copolymers is particularly preferred.

The (acrylic acid/C10-30 alkyl acrylate) copolymer is obtained by crosslinking a copolymer of C10-30 alkyl acrylate with acrylic acid, methacrylic acid, or a lower alkyl ester thereof, with an allyl ether of sucrose or an allyl ether of pentaerythritol. Commercial products, such as PEMULEN TR-1 and PEMULEN TR-2 (both of which are manufactured by Lubrizol), can be used.

Acidic aqueous thickeners, such as polyacrylic acid, carboxyvinyl polymers, and (acrylic acid/C10-30 alkyl acrylate) copolymers, may be used as water-soluble or water-dispersible salts using an alkali metal hydroxide, such as potassium hydroxide or sodium hydroxide, as a neutralizing agent.

Among these components (a5), in terms of the uniformity of the coating film, UV protection efficacy, storage stability, and the like, it is preferable to use at least an oily thickener, more preferably a combination of an aqueous thickener and an oily thickener, further more preferably a combination of an aqueous thickener and a sugar fatty acid ester-based oily thickener, and particularly preferably a combination of one or more selected from the group consisting of polyvinyl alcohol and (meth)acrylic acid-based polymers, and a sugar fatty acid ester-based oily thickener.

When at least an oily thickener is used as the component (a5), the uniformity of the coating film can be further improved. When a UV absorber is used in combination, the UV absorber is likely to be uniformly distributed, and UV protection efficacy is particularly improved.

When an aquoues thickener and an oily thickener are used in combination, the content mass ratio of the aqueous thickener to the oily thickener, [(aqueous thickener)/(oily thickener)], is preferably 0.01 or more, more preferably 0.05 or more, further more preferably 0.1 or more, and particularly preferably 0.3 or more, in terms of the emulsion stability of the capsules, storage stability, and the like. Further, in terms of fresh feeling at the beginning of application, watery feeling at the beginning of application, storage stability, and the like, the content mass ratio is preferably 1.5 or less, more preferably 1 or less, further more preferably 0.75 or less, and particularly preferably 0.5 or less. The specific range is preferably 0.01 or more and 1.5 or less, more preferably 0.05 or more and 1 or less, further more preferably 0.1 or more and 0.75 or less, and particularly preferably 0.3 or more and 0.5 or less.

The thickeners may be used singly or in combinations of two or more thereof.

When the granular gel capsules contain an encapsulating agent and a aqueous thickener, the aqueous thickener forms a uniform coating film, together with the non-volatile oil agent, using the encapsulating agent as a scaffold upon the collapse of the granular gel capsules during application, ensuring good adhesive feeling in the later stages of application. When a UV absorber is used in combination, UV protection efficacy is particularly improved.

The content of the oily thickener in the granular gel capsules is preferably 0.05 mass% or more, more preferably 0.1 mass% or more, further more preferably 0.3 mass% or more, and particularly preferably 0.5 mass% or more, based on the total mass of the dermatological topical agent of the present invention, in terms of UV protection efficacy, the emulsion stability of the capsules, and the like. Further, in terms of fresh feeling at the beginning of application, no stickiness during application, and the like, the content of the oily thickener is preferably 12 mass% or less, more preferably 9 mass% or less, further more preferably 6 mass% or less, and particularly preferably 2 mass% or less, based on the total mass of the dermatological topical agent of the present invention. The specific range is preferably 0.05 mass% or more and 12 mass% or less, more preferably 0.1 mass% or more and 9 mass% or less, further more preferably 0.3 mass% or more and 6 mass% or less, and particularly preferably 0.5 mass% or more and 2 mass% or less, based on the total mass of the dermatological topical agent of the present invention.

The content ratio of the oily thickener in the granular gel capsules is preferably 0.1 mass% or more, more preferably 0.5 mass% or more, further more preferably 1 mass% or more, and particularly preferably 1.5 mass% or more, based on the total mass of the granular gel capsules, in terms of UV protection efficacy, the emulsion stability of the capsules, and the like. Further, in terms of fresh feeling at the beginning of application, no stickiness during application, and the like, the content ratio of the oily thickener is preferably 10 mass% or less, more preferably 7.5 mass% or less, further more preferably 5 mass% or less, and particularly preferably 3.5 mass% or less, based on the total mass of the granular gel capsules. The specific range is preferably 0.1 mass% or more and 10 mass% or less, more preferably 0.5

mass% or more and 7.5 mass% or less, further more preferably 1 mass% or more and 5 mass% or less, and particularly preferably 1.5 mass% or more and 3.5 mass% or less, based on the total mass of the granular gel capsules.

In the granular gel capsules, the content mass ratio of the component (a5) thickener to the component (a1) UV absorber (preferably an oil-soluble UV absorber), [(a5)/(a1)], is preferably 0.005 or more, more preferably 0.01 or more, further more preferably 0.03 or more, and particularly preferably 0.05 or more, in terms of the emulsion stability of the capsules, storage stability, and the like. Further, in terms of fresh feeling at the beginning of application, no stickiness during application, and the like, the content mass ratio is preferably 1 or less, more preferably 0.5 or less, further more preferably 0.3 or less, and particularly preferably 0.1 or less. The specific range is preferably 0.005 or more and 1 or less, more preferably 0.01 or more and 0.5 or less, further more preferably 0.03 or more and 0.3 or less, and particularly preferably 0.05 or more and 0.1 or less.

The content of the aqueous thickener (including polyvinyl alcohol) in the granular gel capsules is preferably 0.01 mass% or more, more preferably 0.05 mass% or more, and further more preferably 0.1 mass% or more, based on the total mass of the dermatological topical agent of the present invention, in terms of the emulsion stability of the capsules and gel strength. The content of the aqueous thickener (including polyvinyl alcohol) in the granular gel capsules is preferably 2.0 mass% or less, more preferably 1.5 mass% or less, and further more preferably 1.0 mass% or less, in terms of fresh feeling at the beginning of application, no stickiness during application, and the like.

The content of the aqueous thickener (including polyvinyl alcohol) in the entire dermatological topical agent is preferably 0.6 mass% or less, more preferably 0.55 mass% or less, further more preferably 0.5 mass% or less, and even more preferably 0.3 mass% or less, based on the total mass of the dermatological topical agent of the present invention, in terms of the formation of a uniform coating film, adhesive feeling in the later stages of application, and the like. The content of the aqueous thickener other than polyvinyl alcohol in the entire dermatological topical agent is preferably 0.3 mass% or less, more preferably 0.25 mass% or less, further more preferably 0.13 mass% or less, even more preferably 0.06 mass% or less, and even more preferably 0.03 mass% or less, based on the total mass of the dermatological topical agent of the present invention, in terms of the formation of a uniform coating film, adhesive feeling in the later stages of application, and the like.

((a6) Polyhydric Alcohol)

Examples of polyhydric alcohols include glycols, glycerins, and the like. Examples of glycols include alkylene glycols, such as ethylene glycol, propylene glycol, and 1,3-butylene glycol; dialkylene glycols, such as diethylene glycol and dipropylene glycol; and polyalkylene glycols, such as polyethylene glycol and polypropylene glycol. Examples of glycerins include glycerin, diglycerin, polyglycerin, and the like.

Among these, in terms of storage stability (stabilization of the granular gel capsules), smoothness during application, and the like, glycerins are preferred, and glycerin is particularly preferred. When glycerins are used as the components (a6), excellent storage stability can be achieved even though the high content of the non-volatile oil agent (preferably an oil-soluble UV absorber) in the granular gel capsules.

The polyhydric alcohols may be used singly or in combinations of two or more thereof.

The content of the polyhydric alcohol in the granular gel capsules is preferably 0 mass% or more, more preferably 1 mass% or more, further more preferably 2 mass% or more, and particularly preferably 3 mass% or more, based on the total mass of the dermatological topical agent of the present invention, in terms of storage stability (stabilization of the granular gel capsules) and the like. Further, in terms of no stickiness during application and the like, the content of the polyhydric alcohol is preferably 16 mass% or less, more preferably 14 mass% or less, further more preferably 12 mass% or less, and particularly preferably 10 mass% or less, based on the total mass of the dermatological topical agent of the present invention. The specific range is preferably 0 mass% or more and 16 mass% or less, more preferably 1 mass% or more and 14 mass% or less, further more preferably 2 mass% or more and 12 mass% or less, and particularly preferably 3 mass% or more and 10 mass% or less, based on the total mass of the dermatological topical agent of the present invention.

The content ratio of the polyhydric alcohol in the granular gel capsules is preferably 0 mass% or more, more preferably 1 mass% or more, further more preferably 3 mass% or more, and particularly preferably 10 mass% or more, based on the total mass of the granular gel capsules, in terms of storage stability (stabilization of the granular gel capsules) and the like. Further, in terms of no stickiness during application and the like, the content ratio of the polyhydric alcohol is preferably 30 mass% or less, more preferably 26 mass% or less, further more preferably 22 mass% or less, and particularly preferably 18 mass% or less, based on the total mass of the granular gel capsules. The specific range is preferably 0 mass% or more and 30 mass% or less, more preferably 1 mass% or more and 26 mass% or less, further more preferably 3 mass% or more and 22 mass% or less, and particularly preferably 10 mass% or more and 18 mass% or less, based on the total mass of the granular gel capsules.

In the granular capsules, the content mass ratio of the component (a6) polyhydric alcohol to the component (a1) UV absorber (preferably an oil-soluble UV absorber), [(a6)/(a1)], is preferably 0 or more, more preferably 0.02 or

more, further more preferably 0.05 or more, and particularly preferably 0.2 or more, in terms of storage stability (stabilization of the granular gel capsules) and the like. Further, in terms of no stickiness during application and the like, the content mass ratio is preferably 1.8 or less, more preferably 1.4 or less, further more preferably 1 or less, and particularly preferably 0.6 or less. The specific range is preferably 0 or more and 1.8 or less, more preferably 0.02 or more and 1.4 or less, further more preferably 0.05 or more and 1 or less, and particularly preferably 0.2 or more and 0.6 or less.

((a7) Powder)

Examples of the powder include UV scattering agents, powders other than UV scattering agents, and the like. These may be used singly or in combinations of two or more thereof.
Among these, the powder to be contained in the granular gel capsules is preferably a UV scattering agent, in terms of UV protection efficacy and the like.
Examples of the UV scattering agent include metal oxides, such as titanium oxide, zinc oxide, and cerium oxide; metals, such as silicon and aluminum. Among these, metal oxides are preferred, and titanium oxide is particularly preferred.
The form of the UV scattering agent is not particularly limited, and is, for example, spherical, plate-like, rod-like, spindle-like, needle-like, or irregular.
The average primary particle size of the UV scattering agent is preferably from 0.005 to 0.5 μm, more preferably from 0.007 to 0.2 μm, and particularly preferably from 0.01 to 0.07 μm. The average primary particle size of the UV scattering agent is determined by observing the UV scattering agent with a transmission electron microscope (TEM) at a magnification of 100 000 times, measuring the maximum minor axes of 300 primary particles in the observed image, and calculating the number average value thereof. The maximum minor axis means the longest minor axis among the minor axes perpendicular to the major axes when the UV scattering agent has a shape other than a plate. When the UV scattering agent has a plate-like shape, the average primary particle size is obtained by measuring the thickness of 300 primary particles in an observed image observed under the same conditions as above and calculating the number average value thereof.
The UV scattering agent is preferably a hydrophobized UV scattering agent.
Examples of hydrophobic treatment include fluorine compound treatments, such as perfluoroalkyl phosphate ester treatment, perfluoroalkylsilane treatment, perfluoropolyether treatment, fluorosilicone treatment, and fluorinated silicone resin treatment; silicone treatments, such as methylhydrogenpolysiloxane treatment, dimethylpolysiloxane treatment, and vapor-phase tetramethyltetrahydrogencyclotetrasiloxane treatment; silicone resin treatments, such as trimethylsiloxysilicate treatment; pendant treatment (a method of adding an alkyl chain or the like after vapor-phase silicone treatment); silane coupling agent treatment; titanium coupling agent treatment; silane treatments, such as alkylsilane treatment, alkylalkoxysilane treatment (e.g., triethoxycaprylylsilane treatment), fluoroalkylalkoxysilane treatment, and alkylsilazane treatment; oil agent treatment; N-acylated lysine treatment; polyacrylic acid treatment; metal soap treatments, such as stearate treatment and myristate treatment; acrylic resin treatment; metal oxide treatments, such as hydrous silica treatment; and the like. A plurality of these treatments may be used in combination. For example, the surface of titanium oxide is coated with silane, alumina, or the like, followed by surface treatment with alkylsilane or fatty acid.
Among these hydrophobic treatments, silicone treatments, silane treatments, metal soap treatments, metal oxide treatments, and the like are preferred.
The powder other than UV scattering agents may be an organic powder, an inorganic powder, or an organic-inorganic composite powder. Examples thereof include hydrophobized talc, cellulose, silica (specifically hydrophilic/hydrophobic, porous/non-porous silica), crosslinked (meth)acrylic acid ester-based resins, silicone resins, and the like. These may be used singly or in combinations of two or more thereof.
The average particle size of the powder other than UV scattering agents is preferably from 0.1 to 30 μm, more preferably from 0.5 to 25 μm, and particularly preferably from 1 to 20 μm.
The average particle size of the powder other than UV scattering agents refers to a volume-based median diameter measured using a laser scattering particle size distribution analyzer LA-960 (manufactured by HORIBA).
The powders may be used singly or in combinations of two or more thereof.
The content ratio of the powder in the granular gel capsules is preferably 0 mass% or more and 40 mass% or less, more preferably 0 mass% or more and 30 mass% or less, further more preferably 0 mass% or more and 20 mass% or less, and particularly preferably 0 mass% or more and 10 mass% or less, based on the total mass of the granular gel capsules, in terms of fresh feeling at the beginning of application, watery feeling at the beginning of application, production efficiency, and the like. Even with such a low content ratio of the powder in the granular gel capsules, the present invention ensures excellent UV protection efficacy.

((a8) Non-Volatile Oil Agent (Excluding Oil-Soluble UV Absorbers, Amphiphilic Solid Fats, and Oily Thickeners))

Examples of the component (a8) non-volatile oil agent include non-volatile hydrocarbon oils, such as hydrogenated polyisobutene, fluid paraffin, and vaseline; non-volatile silicone oils, such as non-volatile dimethylpolysiloxane; non-volatile fatty acid ester oils, such as isopropyl myristate, isopropyl palmitate, tri(caprylic acid/capric acid) glyceryl, isononyl isononanoate, esters of fatty acids and neopentyl glycol (e.g., neopentyl glycol dicaprate); C12-15 alkyl benzoate; and the like. Non-volatile oil agents also include non-volatile animal oils and non-volatile vegetable oils (e.g., olive fruit oil).
The component (a8) non-volatile oil agents may be used singly or in combinations of two or more thereof.
The content ratio of the component (a8) non-volatile oil agent in the granular gel capsules is preferably 0 mass% or more and 50 mass% or less, more preferably 0 mass% or more and 30 mass% or less, further more preferably 0 mass% or more and 15 mass% or less, and particularly preferably 0 mass% or more and 5 mass% or less, based on the total mass of the granular gel capsules.
In addition to the above components, the granular gel capsules may also contain an oil agent (excluding oil-soluble UV absorbers, amphiphilic solid fats, oily thickeners, and the component (a8)), a chelating agent, a dispersant, a preservative, and the like. These may be used singly or in combinations of two or more thereof.
The average particle size of the granular gel capsules is preferably 20 μm. or more, more preferably 30 μm or more, further more preferably 40 μm or more, further more preferably 60 μm. or more, and particularly preferably 80 μm. or more, in terms of storage stability (stabilization of the granular gel capsules), use impression, production efficiency, easy collapse of the granular gel capsules during application, and the like. Further, in terms of use impression during application and the like, the average particle size of the granular gel capsules is preferably 350 μm. or less, more preferably 300 μm or less, further more preferably 250 μm. or less, and particularly preferably 200 μm. or less. The specific range of the average particle size is preferably 20 μm. or more and 350 μm. or less, more preferably 30 μm or more and 300 μm or less, further more preferably 40 μm or more and 300 μm or less, further more preferably 60 μm or more and 250 μm or less, and particularly preferably 80 μm or more and 200 μm or less.
With an average particle size of the granular gel capsules of 60 μm or more, storage stability (stability of the granular gel capsules) is particularly improved.
The average particle size of the granular gel capsules refers to a volume-based median diameter with a refractive index of 1.20 measured by using a laser scattering particle size distribution analyzer LA-960 (manufactured by HORIBA).
The content of the granular gel capsules is preferably 15 mass% or more, more preferably 20 mass% or more, further more preferably 22.5 mass% or more, further more preferably 27.5 mass% or more, further more preferably 30 mass% or more, further more preferably 35 mass% or more, and particularly preferably 40 mass% or more, based on the total mass of the dermatological topical agent of the present invention, in terms of UV protection efficacy and the like. Further, in terms of handling properties, spreading while applying, and the like, the content of the granular gel capsules is preferably 80 mass% or less, more preferably 75 mass% or less, further more preferably 72.5 mass% or less, and particularly preferably 70 mass% or less, based on the total mass of the dermatological topical agent of the present invention. The specific range is preferably 15 mass% or more and 80 mass% or less, more preferably 20 mass% or more and 75 mass% or less, further more preferably 22.5 mass% or more and 75 mass% or less, further more preferably 27.5 mass% or more and 72.5 mass% or less, further more preferably 30 mass% or more and 72.5 mass% or less, further more preferably 35 mass% or more and 70 mass% or less, and particularly preferably 40 mass% or more and 70 mass% or less, based on the total mass of the dermatological topical agent of the present invention.

<(B) Liquid>

The liquid in which the granular gel capsules are dispersed contains (b1) a volatile medium. Even when the granular gel capsules contain a volatile medium, the volatile medium (b1) refers to a volatile medium outside the granular gel capsules.
In the present specification, liquids in which "granular gel capsules are dispersed" include liquids in which some or all of the granular gel capsules are precipitated, as long as some or all of them can be redispersed by stirring or shaking.

((b1) Volatile Medium)

Examples of the volatile medium (b1) contained in the component (B) liquid include water and lower alcohols. Examples of lower alcohols include ethanol, isopropanol, and the like.
The volatile media may be used singly or in combinations of two or more thereof.
The content of the volatile medium (b1) in the component (B) liquid is preferably 20 mass% or more, more preferably 23 mass% or more, further more preferably 26 mass% or more, and particularly preferably 30 mass% or more, based on the total mass of the dermatological topical agent of the present invention, in terms of fresh feeling at the beginning of application, watery feeling at the beginning of application, and the like. The content of the volatile

medium (b1) is also preferably 70 mass% or less, more preferably 65 mass% or less, further more preferably 60 mass% or less, and particularly preferably 55 mass% or less, based on the total mass of the dermatological topical agent of the present invention. The specific range is preferably 20 mass% or more and 70 mass% or less, more preferably 23 mass% or more and 65 mass% or less, further more preferably 26 mass% or more and 60 mass% or less, and particularly preferably 30 mass% or more and 55 mass% or less, based on the total mass of the dermatological topical agent of the present invention.

The content ratio of the volatile medium (b1) in the component (B) liquid is preferably 60 mass% or more, more preferably 75 mass% or more, further more preferably 85 mass% or more, and particularly preferably 95 mass% or more, based on the total mass of the component (B) liquid, in terms of fresh feeling at the beginning of application, watery feeling at the beginning of application, and the like. The content ratio of the volatile medium (b1) is also preferably 100 mass% or less based on the total mass of the component (B) liquid. The specific range is preferably 60 mass% or more and 100 mass% or less, more preferably 75 mass% or more and 100 mass% or less, further more preferably 85 mass% or more and 100 mass% or less, and particularly preferably 95 mass% or more and 100 mass% or less, based on the total mass of the component (B) liquid.

When water is used as the component (b1), the content ratio of water in the component (B) liquid is preferably 40 mass% or more, more preferably 50 mass% or more, further more preferably 55 mass% or more, and particularly preferably 65 mass% or more, based on the total mass of the component (B) liquid, in terms of fresh feeling at the beginning of application, watery feeling at the beginning of application, and the like. The content ratio of water is also preferably 98 mass% or less, more preferably 95 mass% or less, further more preferably 92 mass% or less, and particularly preferably 88 mass% or less, based on the total mass of the component (B) liquid. The specific range is preferably 40 mass% or more and 98 mass% or less, more preferably 50 mass% or more and 95 mass% or less, further more preferably 55 mass% or more and 92 mass% or less, and particularly preferably 65 mass% or more and 88 mass% or less, based on the total mass of the component (B) liquid.

When a lower alcohol is used as the component (b1), the content ratio of the lower alcohol in the component (B) liquid is preferably 2 mass% or more, more preferably 4 mass% or more, further more preferably 8 mass% or more, and particularly preferably 10 mass% or more, based on the total mass of the component (B) liquid, in terms of fresh feeling at the beginning of application, watery feeling at the beginning of application, and the like. Further, in terms of storage stability and the like, the content ratio of the lower alcohol is preferably 46 mass% or less, more preferably 42 mass% or less, further more preferably 38 mass% or less, and particularly preferably 35 mass% or less, based on the total mass of the component (B) liquid. The specific range is preferably 2 mass% or more and 46 mass% or less, more preferably 4 mass% or more and 42 mass% or less, further more preferably 8 mass% or more and 38 mass% or less, and particularly preferably 10 mass% or more and 35 mass% or less, based on the total mass of the component (B) liquid.

((b2) Cationic Polymer)

The component (B) liquid preferably further contains (b2) a cationic polymer in addition to the volatile medium (b1). Even when the granular gel capsules contain a cationic polymer, the cationic polymer (b2) refers to a cationic polymer outside the granular gel capsules.

In general, when silica is incorporated into a dermatological topical agent, its use impression is likely to be less sticky; however, caking tends to occur easily. When the dermatological topical agent of the present invention contains silica as a powder as a component (C) in addition to the granular gel capsules (A) and the liquid (B), and when the component (B) liquid contains (b2) a cationic polymer, use impression and redispersibility (difficulty in caking) can both be achieved.

The cationic polymer is preferably a silicone-based cationic polymer. Examples thereof include poly(N-acylalkyleneimine)-modified silicone (e.g., oxazoline-modified silicone), amino-modified silicone, and the like. These may be used singly or in combinations of two or more thereof.

Among these, poly(N-acylalkyleneimine)-modified silicone is preferred, in terms of making it difficult for caking to occur.

Examples of poly(N-acylalkyleneimine)-modified silicone include organopolysiloxane in which poly(N-acylalkyleneimine) segments containing repeating units of the following formula (11) are bonded via cationic divalent linking groups to at least two of the silicon atoms of an organopolysiloxane segment constituting the main chain (hereinafter, this organopolysiloxane is also referred to as organopolysiloxane (OX)).

At least two poly(N-acylalkyleneimine) segments are bonded to arbitrary silicon atoms constituting the organopolysiloxane segment via cationic divalent linking groups. Bonding to one or more silicon atoms excluding both ends of the organopolysiloxane segment via a cationic divalent linking group is preferred, and bonding to two or more silicon atoms excluding both ends via cationic divalent linking groups is more preferred.

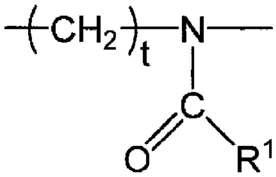
(11)

[In the formula (11), $R^{11}$ represents a hydrogen atom, an alkyl group having from 1 to 22 carbon atoms, an aralkyl group, or an aryl group, and t represents 2 or 3.]

The cationic divalent linking group serves as a linking group for the poly(N-acylalkyleneimine) segments.

Examples of the cationic divalent linking group include alkylene groups having from 2 to 20 carbon atoms containing one to three cationic groups of one or more selected from the group consisting of primary amino groups, secondary amino groups, tertiary amino groups, and quaternary ammonium groups. Specific examples thereof include a group of any of the following formulas (A1) to (A9), preferably a group of any of the formulas (A1) to (A4), and particularly preferably a group of the formula (A1) or (A2).

In the formulas, $An^-$ represents a counter ion of a quaternary ammonium salt. Examples thereof include halide ions (e.g., chloride ions and iodide ions), sulfate ions, phosphate ions, acetate ions, lactate ions, p-toluenesulfonate ions, perchlorate ions, monoalkyl nitrate ions (e.g., methyl sulfate ions and ethyl sulfate ions), and the like.

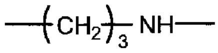
(A1)

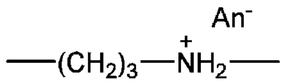
(A2)

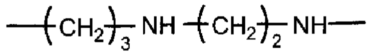
(A3)

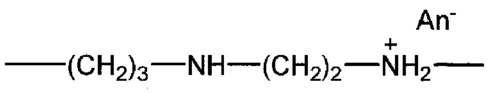
(A4)

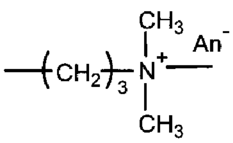
(A5)

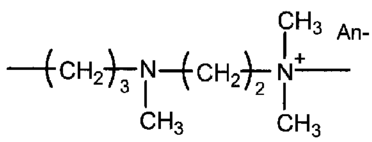
(A6)

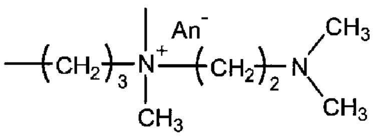
(A7)

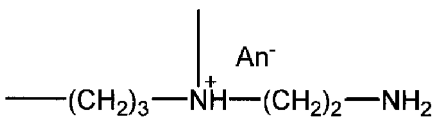
(A8)

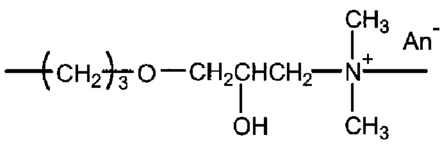
(A9)

In the N-acylalkyleneimine unit constituting the poly(N-acylalkyleneimine) segment, the number of carbon atoms in the alkyl group of $R^{11}$ in the formula (11) is from 1 to 22, preferably from 1 to 14, more preferably from 1 to 6, and

particularly preferably from 1 to 3. The above alkyl group may be linear or branched. Examples thereof include methyl, ethyl, n-propyl, isopropyl, n-butyl, tert-butyl, pentyl, and hexyl groups.

The aralkyl group of $R^{11}$ is preferably an aralkyl group having from 7 to 15 carbon atoms. Examples thereof include benzyl, phenethyl, trityl, naphthylmethyl, anthracenylmethyl groups.

The aryl group of $R^{11}$ is preferably an aryl group having from 6 to 14 carbon atoms. Examples thereof include phenyl, tolyl, xylyl, naphthyl, biphenyl, anthracenyl, and phenanthryl groups.

Among these, $R^{11}$ is preferably a hydrogen atom or a linear or branched alkyl group having from 1 to 3 carbon atoms, and more preferably a linear or branched alkyl group having from 1 to 3 carbon atoms.

t in the formula (11) represents 2 or 3, and t is preferably 2.

In the organopolysiloxane (OX), the weight average molecular weight (hereinafter also referred to as "MWg") of the organopolysiloxane segment between adjacent poly(N-acylalkyleneimine) segments is preferably from 1 000 to 40 000, and more preferably from 1 500 to 30 000.

The molecular weight of the poly(N-acylalkyleneimine) segment can be calculated from the molecular weight of the N-acylalkyleneimine unit and the degree of polymerization, or can be measured by gel permeation chromatography (GPC); however, in the present specification, this molecular weight refers to a polystyrene-equivalent number average molecular weight (hereinafter also referred to as MNox) measured by GPC under the measurement conditions described later. MNox is preferably from 500 to 4 000.

MWg can also be determined by the following equation (I) using the content (mass%) of the organopolysiloxane segment constituting the main chain (hereinafter also referred to as Csi).

$$MWg = Csi \times MNox/(100 - Csi) \quad (I)$$

The weight average molecular weight of the organopolysiloxane segment constituting the main chain (hereinafter also referred to as MWsi) is preferably from 10 000 to 200 000, and more preferably from 15 000 to 160 000.

Since the organopolysiloxane segment constituting the main chain has a skeleton in common with modified organopolysiloxane as a raw material compound, MWsi is approximately the same as the weight average molecular weight of the modified organopolysiloxane as the raw material compound. The weight average molecular weight of the modified organopolysiloxane as the raw material compound is a polystyrene-equivalent weight average molecular weight measured by GPC under the following measurement conditions.

(Measurement conditions of weight average molecular weight of modified organopolysiloxane)

Columns: Super HZ4000 + Super HZ2000 (manufactured by Tosoh Corporation)
Eluent: 1 mM triethylamine/THF
Flow rate: 0.35 mL/min
Column temperature: 40°C
Detector: UV
Sample: 50 μL

The weight average molecular weight (hereinafter also referred to as MWt) of the organopolysiloxane (OX) is preferably from 10 000 to 500 000, and more preferably from 12 000 to 200 000. MWt refers to a polystyrene-equivalent value measured by GPC under the measurement conditions described later.

(Measurement conditions of MNox and MWt)

Columns: two K-804L (manufactured by Tosoh Corporation) columns connected in series for use
Eluent: 1 mM dimethyldodecylamine/chloroform
Flow rate: 1.0 mL/min
Column temperature: 40°C
Detector: RI
Sample: 50 μL

The mass ratio of the organopolysiloxane segment constituting the main chain (a) and the poly(N-acylalkyleneimine) segment (b), (a/b), is preferably from 40/60 to 98/2, and more preferably from 45/55 to 82/18.

In the present specification, the mass ratio (a/b) refers to a value determined in such a manner that 5 mass% of organopolysiloxane (OX) is dissolved in heavy chloroform, and the integral ratio of the alkyl or phenyl groups in the organopolysiloxane segment, and the methylene groups in the poly(N-acylalkyleneimine) segment is determined by nuclear magnetic resonance ($^1$H-NMR) analysis.

When the poly(N-acylalkyleneimine) segment is a poly (N-propionylethyleneimine) segment, the $^1$H-NMR measurement for the calculation of the mass ratio (a/b) can be performed, for example, under the following conditions.

($^1$H-NMR measurement conditions)

A polymer sample (0.5 g) is dissolved in 2 g of a measurement solvent (heavy chloroform) and measured by $^1$H-NMR (400 MHz, manufactured by Varian). Then, the ratio of silicone and poly(N-propionylethyleneimine) is calculated from each integrated value.

PULSE SEQUENCE

- Relax. delay: 30 sec - Pulse: 45 degrees - Number of integration: 8 times

Confirmation peak around 0 ppm: the methyl group of polydimethylsiloxane,
around 3.4 ppm: the methylene moiety of ethyleneimine.

The organopolysiloxane (OX) may be synthesized in accordance with the method described in JP-A-2008-143820, JP-A-2009-24114, JP-A-2015-67603, JP-A-2016-204336, or the like. For example, the organopolysiloxane (OX) can be produced by reacting an organopolysiloxane modified with a functional group which induces the above cationic divalent linking group, and terminal reactive poly(N-acylalkyleneimine) obtained by ring-opening polymerization of a cyclic imino ether corresponding to the repeating unit of the formula (11).

Specific examples of poly(N-acylalkyleneimine)-modified silicone include those prepared in accordance with the disclosure of Synthesis Examples 1 and 2 in JP-A-2008-143820, and those prepared in accordance with the disclosure of Example 8 in JP-A-2009-24114.

The cationic polymers may be used singly or in combinations of two or more thereof.

The content of the cationic polymer (b2) in the component (B) liquid is preferably 0 mass% or more, more preferably 0.005 mass% or more, further more preferably 0.01 mass% or more, further more preferably 0.02 mass% or more, and particularly preferably 0.05 mass% or more, based on the total mass of the dermatological topical agent of the present invention, in terms of redispersibility (difficulty in caking), durability of UV protection efficacy, and the like. Further, in terms of no stickiness during application and the like, the content of the cationic polymer (b2) is preferably 1 mass% or less, more preferably 0.6 mass% or less, further more preferably 0.3 mass% or less, and particularly preferably 0.1 mass% or less, based on the total mass of the dermatological topical agent of the present invention. The specific range is preferably 0 mass% or more and 1 mass% or less, more preferably 0.005 mass% or more and 0.6 mass% or less, further more preferably 0.01 mass% or more and 0.3 mass% or less, and particularly preferably 0.02 mass% or more and 0.1 mass% or less, based on the total mass of the dermatological topical agent of the present invention.

The content ratio of the cationic polymer (b2) in the component (B) liquid is preferably 0 mass% or more, more preferably 0.01 mass% or more, further more preferably 0.05 mass% or more, and particularly preferably 0.1 mass% or more, based on the total mass of the component (B) liquid, in terms of redispersibility (difficulty in caking), durability of UV protection efficacy, and the like. Further, in terms of no stickiness during application and the like, the content ratio of the cationic polymer (b2) is preferably 3 mass% or less, more preferably 1 mass% or less, further more preferably 0.75 mass% or less, and particularly preferably 0.5 mass% or less, based on the total mass of the component (B) liquid. The specific range is preferably 0 mass% or more and 3 mass% or less, more preferably 0.01 mass% or more and 1 mass% or less, further more preferably 0.05 mass% or more and 0.75 mass% or less, and particularly preferably 0.1 mass% or more and 0.5 mass% or less, based on the total mass of the component (B) liquid.

The component (B) liquid may contain, in addition to the above components, a disinfectant, a moisturizer, and the like. These may be used singly or in combinations of two or more thereof.

The total content of the component (B) liquid is preferably 20 mass% or more, more preferably 24 mass% or more, further more preferably 27 mass% or more, and particularly preferably 30 mass% or more, based on the total mass of the dermatological topical agent of the present invention, in terms of handling properties and the like. The total content of the component (B) liquid is also preferably 85 mass% or less, more preferably 75 mass% or less, further more preferably 70 mass% or less, and particularly preferably 65 mass% or less, based on the total mass of the dermatological topical agent of the present invention. The specific range is preferably 20 mass% or more and 85 mass% or less, more preferably 24 mass% or more and 75 mass% or less, further more preferably 27 mass% or more and 70 mass% or less, and particularly preferably 30 mass% or more and 65 mass% or less, based on the total mass of the dermatological

topical agent of the present invention.

The content mass ratio of the component (A) granular gel capsules to the component (B) liquid, [(A)/(B)], is preferably 0.1 or more, more preferably 0.2 or more, further more preferably 0.4 or more, and particularly preferably 0.5 or more, in terms of the uniformity of the coating film, UV protection efficacy, and the like. Further, in terms of fresh feeling at the beginning of application, watery feeling at the beginning of application, and the like, the content mass ratio is preferably 3.5 or less, more preferably 3 or less, further more preferably 2.5 or less, and particularly preferably 2 or less. The specific range is preferably 0.1 or more and 3.5 or less, more preferably 0.2 or more and 3 or less, further more preferably 0.4 or more and 2.5 or less, and particularly preferably 0.5 or more and 2 or less.

The content of the non-volatile oil agent in the component (B) liquid is 0 mass% or more and 14 mass% or less based on the total mass of the dermatological topical agent of the present invention. By thus setting the content of the non-volatile oil agent to 0 mass% or more and 14 mass% or less, fresh and smooth use impression can be obtained at the beginning of application.

The content of the non-volatile oil agent in the component (B) liquid is preferably 0 mass% or more and 10 mass% or less, more preferably 0 mass% or more and 5 mass% or less, further more preferably 0 mass% or more and 1 mass% or less, and particularly preferably 0 mass%, based on the total mass of the dermatological topical agent of the present invention, in terms of fresh feeling at the beginning of application, smoothness during application, and the like.

The content ratio of the non-volatile oil agent in the component (B) liquid is preferably 0 mass% or more and 25 mass% or less, more preferably 0 mass% or more and 15 mass% or less, further more preferably 0 mass% or more and 5 mass% or less, and particularly preferably 0 mass%, based on the total mass of the component (B) liquid, in terms of fresh feeling at the beginning of application, watery feeling at the beginning of application, and the like.

The content mass ratio of all non-volatile oil agents in the (B) liquid to the total of all non-volatile oil agents in the granular gel capsules, [(all non-volatile oil agents in liquid)/(all non-volatile oil agents in granular gel capsules)], is preferably 0 or more and 0.1 or less, more preferably 0 or more and 0.06 or less, further more preferably 0 or more and 0.01 or less, and particularly preferably 0, in terms of fresh feeling at the beginning of application, watery feeling at the beginning of application, and the like.

The "non-volatile oil agents" mentioned above refer to oily components which are not volatile at 25°C under 1 atm. For example, non-volatile oil-soluble UV absorbers, non-volatile amphiphilic solid fats, and non-volatile oily thickeners are non-volatile oil agents. Further, examples of non-volatile oil agents include non-volatile hydrocarbon oils, such as hydrogenated polyisobutene, fluid paraffin, and vaseline; non-volatile silicone oils, such as non-volatile dimethylpolysiloxane; non-volatile fatty acid ester oils, such as isopropyl myristate, isopropyl palmitate, tri(caprylic acid/capric acid) glyceryl, isononyl isononanoate, esters of fatty acids and neopentyl glycol (e.g., neopentyl glycol dicaprate); C12-15 alkyl benzoate; and the like. Non-volatile oil agents also include non-volatile animal oils and non-volatile vegetable oils (e.g., olive fruit oil).

The content of the aqueous thickener in the component (B) liquid is 0 mass% or more and 0.25 mass% or less based on the total mass of the dermatological topical agent of the present invention. By thus setting the content of the aqueous thickener to 0 mass% or more and 0.25 mass% or less, fresh feeling at the beginning of application and watery feeling at the beginning of application are improved.

The content of the aqueous thickener in the component (B) liquid is preferably 0 mass% or more and 0.03 mass% or less, more preferably 0 mass% or more and 0.015 mass% or less, further more preferably 0 mass% or more and 0.005 mass% or less, and particularly preferably 0 mass%, based on the total mass of the dermatological topical agent of the present invention, in terms of fresh feeling at the beginning of application, watery feeling at the beginning of application, and the like.

The content ratio of the aqueous thickener in the component (B) liquid is preferably 0 mass% or more and 0.05 mass% or less, more preferably 0 mass% or more and 0.03 mass% or less, further more preferably 0 mass% or more and 0.01 mass% or less, and particularly preferably 0 mass%, based on the total mass of the component (B) liquid, in terms of fresh feeling at the beginning of application, watery feeling at the beginning of application, and the like.

Examples of the "aqueous thickener" include the same aqueous thickeners as those which may be contained in the granular gel capsules.

The content of the oily thickener in the component (B) liquid is preferably 0 mass% or more and 0.03 mass% or less, more preferably 0 mass% or more and 0.015 mass% or less, further more preferably 0 mass% or more and 0.005 mass% or less, and particularly preferably 0 mass%, based on the total mass of the dermatological topical agent of the present invention, in terms of UV protection efficacy, fresh feeling at the beginning of application, watery feeling at the beginning of application, and the like.

The "oily thickener" mentioned above is a thickener other than aqueous thickeners, and examples thereof include the same oily thickeners as those which may be contained in the granular gel capsules.

The content of an emulsifier in the component (B) liquid is preferably 0 mass% or more and 0.4 mass% or less, more preferably 0 mass% or more and 0.2 mass% or less, further more preferably 0 mass% or more and 0.1 mass% or less, and particularly preferably 0 mass%, based on the total mass of the dermatological topical agent of the present

invention, in terms of fresh feeling at the beginning of application, watery feeling at the beginning of application, emulsion stability, water resistance, and the like.

The content ratio of the emulsifier in the component (B) liquid is preferably 0 mass% or more and 0.8 mass% or less, more preferably 0 mass% or more and 0.4 mass% or less, further more preferably 0 mass% or more and 0.2 mass% or less, and particularly preferably 0 mass%, based on the total mass of the component (B) liquid, in terms of fresh feeling at the beginning of application, watery feeling at the beginning of application, emulsion stability, water resistance, and the like.

Examples of the "emulsifier" include polyoxyethylene hydrogenated castor oil, sorbitan monostearate, and various other surfactants (e.g., anionic surfactants, cationic surfactants, nonionic surfactants, and amphoteric surfactants).

The total content of the non-volatile oil agent, aqueous thickener, and emulsifier in the component (B) liquid is preferably 0 mass% or more and 12 mass% or less, more preferably 0 mass% or more and 5 mass% or less, further more preferably 0 mass% or more and 0.001 mass% or less, and particularly preferably 0 mass%, based on the total mass of the dermatological topical agent of the present invention, in terms of fresh feeling at the beginning of application, watery feeling at the beginning of application, UV protection efficacy, emulsion stability, and the like.

The non-volatile oil agent, aqueous thickener, and emulsifier in the component (B) liquid refer to, respectively, the non-volatile oil agent, aqueous thickener, and emulsifier in the component (B) liquid and outside the granular gel capsules.

<(C) Powder>

In terms of improving use impression and the like, the dermatological topical agent of the present invention is preferably one further containing, in addition to the granular gel capsules (A) and the liquid (B), (C) a powder outside the granular gel capsules, and more preferably one in which the components (A) and (C) are dispersed in the component (B) liquid.

Examples of the component (C) powder include the same powders as those which may be contained in the granular gel capsules, in terms of no sticky feeling, smoothness, and the like; however, a powder other than UV scattering agents is preferred, cellulose and silica are more preferred, silica is further more preferred, and porous silica is particularly preferred.

The average particle size of the powder other than UV scattering agents is preferably from 0.1 to 30 μm, more preferably from 0.5 to 25 μm, and particularly preferably from 1 to 20 μm, in terms of redispersibility (difficulty in caking), UV protection efficacy, no sticky feeling, and the like.

The average particle size of the powder other than UV scattering agents refers to a volume-based median diameter measured using a laser scattering particle size distribution analyzer LA-960 (manufactured by HORIBA).

The content of the component (C) powder is preferably 0.05 mass% or more, more preferably 0.2 mass% or more, further more preferably 0.6 mass% or more, and particularly preferably 1 mass% or more, based on the total mass of the dermatological topical agent of the present invention, in terms of no sticky feeling, smoothness, and the like. Further, in terms of UV protection efficacy, storage stability, and the like, the content of the powder is preferably 15 mass% or less, more preferably 12 mass% or less, further more preferably 9 mass% or less, and particularly preferably 6 mass% or less, based on the total mass of the dermatological topical agent of the present invention. The specific range is preferably 0.05 mass% or more and 15 mass% or less, more preferably 0.2 mass% or more and 12 mass% or less, further more preferably 0.6 mass% or more and 9 mass% or less, and particularly preferably 1 mass% or more and 6 mass% or less, based on the total mass of the dermatological topical agent of the present invention.

When the component (b2) and the component (C) are used in combination, the content mass ratio of the (b2) cationic polymer in the component (B) liquid to the component (C) powder, [(b2)/(C)], is preferably 0.001 or more, more preferably 0.004 or more, further more preferably 0.007 or more, and particularly preferably 0.01 or more, in terms of redispersibility (difficulty in caking), storage stability, durability of UV protection efficacy, suppression of deterioration of the appearance of the dermatological topical agent by emulsifying the oil exuded from the capsules, and the like. Further, in terms of storage stability, no stickiness during application, and the like, the content mass ratio is preferably 1 or less, more preferably 0.5 or less, further more preferably 0.3 or less, and particularly preferably 0.1 or less. The specific range is preferably 0.001 or more and 1 or less, more preferably 0.004 or more and 0.5 or less, further more preferably 0.007 or more and 0.3 or less, and particularly preferably 0.01 or more and 0.1 or less.

The viscosity at 25°C of the dermatological topical agent of the present invention is preferably 10 mPa·s or more, more preferably 20 mPa·s or more, further more preferably 25 mPa·s or more, and particularly preferably 30 mPa·s or more, in terms of difficulty in dripping and the like. Further, in terms of use impression (fresh feeling and watery feeling) at the beginning of application, UV protection efficacy, spreading while applying, and the like, the viscosity at 25°C is preferably 10 000 mPa·s or less, more preferably 5 000 mPa·s or less, further more preferably 1 000 mPa·s or less, and particularly preferably 500 mPa·s or less. The specific range is preferably 10 mPa·s or more and 10 000 mPa·s or less,

more preferably 20 mPa·s or more and 5 000 mPa·s or less, further more preferably 25 mPa·s or more and 1 000 mPa·s or less, and particularly preferably 30 mPa·s or more and 500 mPa·s or less.

With a viscosity at 25°C of 10 000 mPa·s or less, use impression (fresh feeling and watery feeling) at the beginning of application is particularly improved.

The viscosity at 25°C can be measured with a Brookfield-type viscometer (B-type viscometer).

The pH at 25°C of the dermatological topical agent of the present invention is preferably 4 or more and 10 or less, more preferably 4.5 or more and 9 or less, and particularly preferably 5 or more and 8 or less, in terms of storage stability (stabilization of the granular gel capsules) and the like.

With a pH at 25°C of 4.5 or more, storage stability is particularly improved.

The dermatological topical agent of the present invention and the component (B) liquid are in the form of emulsion compositions (oil-in-water type emulsion compositions or water-in-oil type emulsion compositions) or aqueous compositions. In terms of use impression, aqueous compositions are preferred.

The dermatological topical agent of the present invention can be produced by appropriately combining the methods described in JP-A-2012-171891, JP-A-2012-171892, JP-A-2014-91737, JP-A-2016-104712, and the like.

For example, when producing a dermatological topical agent in which granular gel capsules contain an encapsulating agent and water in addition to a non-volatile oil agent, such a dermatological topical agent can be produced in the following manner. Specifically, an encapsulating agent and water are mixed and heated to a temperature equal to or higher than the melting temperature of the encapsulating agent to prepare a water-phase component, and the obtained water-phase component and a separately prepared oil-phase component containing a non-volatile oil agent are mixed to prepare a mixed liquid, followed by dropping, spraying, stirring, or the like, thereby obtaining granular gel capsules. The obtained granular gel capsules are mixed with a volatile medium, whereby a dermatological topical agent can be produced.

The dermatological topical agent of the present invention has excellent use impression, such as fresh feeling at the beginning of application, and can form a good coating film of a non-volatile oil agent, such as an oil-soluble UV absorber.

The present inventor assumes that the reasons for this effect are as follows. By setting the content of the non-volatile oil agent in the granular gel capsules to 1.5 mass% or more based on the total mass of the dermatological topical agent, the content of the non-volatile oil agent in the component (B) liquid to 0 mass% or more and 14 mass% or less based on the total mass of the dermatological topical agent, and the content of the aqueous thickener in the component (B) liquid to 0 mass% or more and 0.25 mass% or less based on the total mass of the dermatological topical agent, good fresh feeling is obtained at the beginning of application, the non-volatile oil agent, such as an oil-soluble UV absorber, is likely to be uniformly distributed, and excellent UV protection efficacy is obtained when the granular gel capsules contain an oil-soluble UV absorber as the non-volatile oil agent. Further, the granular gel capsules collapse during application to form a coating film, which also facilitates good adhesive feeling in the later stages of application, and characteristic use impression, i.e., fresh feeling at the beginning of application and good adhesive feeling in the later stages of application, can be obtained.

Therefore, the dermatological topical agent of the present invention is useful as a cosmetic (e.g., a sunscreen, a foundation, a makeup base, or a skin lotion), and particularly useful as a sunscreen. The dermatological topical agent of the present invention can be used as a sunscreen by applying it to the skin (preferably skin excluding scalp, more preferably face, body, limbs, and the like) by a method in accordance with the type of agent form. The method for use thereof is not particularly limited; however, it is preferable to apply the dermatological topical agent by hand or with an applicator.

The "fresh feeling" mentioned above refers to a feeling of refreshing application like water. For example, when a dermatological topical agent is put on the skin and spread by hand, if it spreads smoothly like water and gives a feeling of refreshing application, this dermatological topical agent is considered to have excellent fresh feeling at the beginning of application. Further, when a dermatological topical agent is applied to the skin, if the dermatological topical agent shows a droplet-like appearance due to the large contact angle of the agent with the skin, the fresh feeling is enhanced.

The adhesive feeling in the later stages of application can be confirmed, for example, by using the inertial sensor described in JP-A-2018-109599 to detect whether the angular velocity Gx in the twisting direction of the finger increases significantly during application.

Regarding the embodiments described above, the present invention further discloses the following dermatological topical agent and the like.

<1> A dermatological topical agent comprising granular gel capsules comprising a non-volatile oil agent, and a liquid comprising the following component (b1):
(b1) a volatile medium,

wherein the granular gel capsules are dispersed in the liquid,

a content of the non-volatile oil agent in the granular gel capsules is 1.5 mass% or more based on a total mass of the dermatological topical agent,
a content of the non-volatile oil agent in the liquid is 0 mass% or more and 14 mass% or less based on the total mass of the dermatological topical agent, and
a content of an aqueous thickener in the liquid is 0 mass% or more and 0.25 mass% or less based on the total mass of the dermatological topical agent.

<2> The dermatological topical agent according to <1>, wherein the granular gel capsules are preferably granular hydrogel capsules, more preferably granular hydrogel capsules in which an oil phase comprising a non-volatile oil agent is dispersed in a hydrogel, further more preferably granular hydrogel capsules in which an oil phase comprising an oil-soluble UV absorber is dispersed in a hydrogel, and particularly preferably granular hydrogel capsules comprising a continuous phase of a non-crosslinked hydrogel and an oil phase dispersed in the continuous phase, the oil phase comprising an oil-soluble UV absorber.
<3> The dermatological topical agent according to <1> or <2>, wherein the granular gel capsules preferably comprise, as the non-volatile oil agent, one or more selected from the group consisting of (a1) a UV absorber (preferably an oil-soluble UV absorber), (a4) an amphiphilic solid fat, (a5-1) an oily thickener, and (a8) a non-volatile oil agent (excluding oil-soluble UV absorbers, amphiphilic solid fats, and oily thickeners); more preferably comprise at least (a1) an oil-soluble UV absorber as the non-volatile oil agent; further more preferably comprise, as the non-volatile oil agents, (a1) an oil-soluble UV absorber and one or more selected from the group consisting of (a4) an amphiphilic solid fat and (a5-1) an oily thickener; and particularly preferably comprise (a1) an oil-soluble UV absorber, (a4) an amphiphilic solid fat, and (a5-1) an oily thickener as the non-volatile oil agents.
<4> The dermatological topical agent according to <3>, wherein the component (a1) is preferably one or more selected from the group consisting of benzoic acid-based UV absorbers, anthranilic acid-based UV absorbers, salicylic acid-based UV absorbers, cinnamic acid-based UV absorbers, benzophenone-based UV absorbers, triazine-based UV absorbers, and silicone-based UV absorbers; more preferably one or more selected from the group consisting of benzoic acid-based UV absorbers, cinnamic acid-based UV absorbers, and triazine-based UV absorbers; and further more preferably one or more selected from the group consisting of para-aminobenzoic acid (PABA), glyceryl PABA, ethyl dihydroxypropyl PABA, N-ethoxylate PABA ethyl ester, N-dimethyl PABA ethyl ester, N-dimethyl PABA butyl ester, N-dimethyl PABA amyl ester, octyl dimethyl PABA, hexyl diethylaminohydroxybenzoylbenzoate, octyl cinnamate, ethyl-4-isopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate, 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, glyceryl mono-2-ethylhexanoyl diparamethoxycinnamate, 2,4,6-tris[4-(2-ethylhexyloxycarbonyl)anilino]-1,3,5-triazine, dioctyl butamido triazone, and 2,4-bis-[{4-(2-ethylhexyloxy)-2-hydroxy}-phenyl]-6-(4-methoxyphenyl)-1,3,5-triazine.
<5> The dermatological topical agent according to <3> or <4>, wherein a content of the component (a1) (preferably an oil-soluble UV absorber) is preferably 1.5 mass% or more, more preferably 3 mass% or more, further more preferably 6 mass% or more, further more preferably 9 mass% or more, and particularly preferably 12 mass% or more, based on the total mass of the dermatological topical agent, and is preferably 40 mass% or less, more preferably 35 mass% or less, further more preferably 30 mass% or less, and particularly preferably 25 mass% or less, based on the total mass of the dermatological topical agent.
<6> The dermatological topical agent according to any one of <3> to <5>, wherein the content of the component (a1) (preferably an oil-soluble UV absorber) is preferably 1.5 mass% or more and 40 mass% or less, more preferably 3 mass% or more and 40 mass% or less, further more preferably 6 mass% or more and 35 mass% or less, further more preferably 9 mass% or more and 30 mass% or less, and particularly preferably 12 mass% or more and 25 mass% or less, based on the total mass of the dermatological topical agent.
<7> The dermatological topical agent according to any one of <3> to <6>, wherein a content ratio of the component (a1) (preferably an oil-soluble UV absorber) is preferably 15 mass% or more, more preferably 17 mass% or more, further more preferably 19 mass% or more, and particularly preferably 22 mass% or more, based on a total mass of the granular gel capsules, and is preferably 54 mass% or less, more preferably 48 mass% or less, further more preferably 42 mass% or less, and particularly preferably 40 mass% or less, based on the total mass of the granular gel capsules.
<8> The dermatological topical agent according to any one of <3> to <7>, wherein a content mass ratio of the UV absorber (preferably an oil-soluble UV absorber) in the component (a1) granular gel capsules to a total of all UV absorbers in the dermatological topical agent, [(a1)/(all UV absorbers)], is preferably 0.6 or more and 1 or less, more preferably 0.85 or more and 1 or less, further more preferably 0.9 or more and 1 or less, further more preferably 0.95 or more and 1 or less, further more preferably 0.99 or more and 1 or less, and particularly preferably 1.
<9> The dermatological topical agent according to any one of <1> to <8>, wherein the granular gel capsules preferably

comprise one or more selected from the group consisting of (a1) a UV absorber (preferably an oil-soluble UV absorber), (a2) an encapsulating agent, (a3) water, (a4) an amphiphilic solid fat, (a5) a thickener (examples of the thickener (a5) include (a5-1) an oily thickener and (a5-2) an aqueous thickener), (a6) polyhydric alcohol, (a7) a powder, and (a8) a non-volatile oil agent (excluding oil-soluble UV absorbers, amphiphilic solid fats, and oily thickeners); more preferably comprise (a1) a UV absorber (preferably an oil-soluble UV absorber); further more preferably further comprise, in addition to (a1) a UV absorber (preferably an oil-soluble UV absorber), one or more selected from the group consisting of (a2) an encapsulating agent, (a3) water, (a4) an amphiphilic solid fat, (a5) a thickener, (a6) polyhydric alcohol, (a7) a powder, and (a8) a non-volatile oil agent (excluding oil-soluble UV absorbers, amphiphilic solid fats, and oily thickeners); further more preferably comprise the components (a1) and (a2); further more preferably comprise the components (a1) to (a3); further more preferably comprise the components (a1) to (a4); further more preferably comprise the components (a1) to (a5); further more preferably comprise the components (a1) to (a5) and one or more selected from the group consisting of the components (a6) and (a7); further more preferably comprise the components (a1) to (a6); and particularly preferably comprise the components (a1) to (a7).

<10> The dermatological topical agent according to <9>, wherein the granular gel capsules are preferably those in which the component (a1) is dispersed in a gel-like encapsulating agent; more preferably those in which the components (a1) and (a3) are dispersed in a gel-like encapsulating agent; further more preferably those in which the components (a1), (a3), and (a4) are dispersed in a gel-like encapsulating agent; further more preferably those in which the component (a1) and the components (a3) to (a5) are dispersed in a gel-like encapsulating agent; further more preferably those in which the component (a1), the components (a3) to (a5), and one or more selected from the group consisting of the components (a6) and (a7) are dispersed in a gel-like encapsulating agent; further more preferably those in which the component (a1) and the components (a3) to (a6) are dispersed in a gel-like encapsulating agent; and particularly preferably those in which the component (a1) and the components (a3) to (a7) are dispersed in a gel-like encapsulating agent.

<11> The dermatological topical agent according to <9> or <10>, wherein the component (a2) is preferably one or more selected from the group consisting of agar, carrageenan, and gelatin, and more preferably agar.

<12> The dermatological topical agent according to any one of <9> to <11>, wherein a content of the component (a2) is preferably 0.05 mass% or more, more preferably 0.1 mass% or more, further more preferably 0.15 mass% or more, and particularly preferably 0.2 mass% or more, based on the total mass of the dermatological topical agent, and is preferably 2 mass% or less, more preferably 1.75 mass% or less, further more preferably 1.5 mass% or less, and particularly preferably 1 mass% or less, based on the total mass of the dermatological topical agent.

<13> The dermatological topical agent according to any one of <9> to <12>, wherein a content ratio of the component (a2) is preferably 0.05 mass% or more, more preferably 0.1 mass% or more, further more preferably 0.2 mass% or more, and particularly preferably 0.4 mass% or more, based on the total mass of the granular gel capsules, and is preferably 10 mass% or less, more preferably 7.5 mass% or less, further more preferably 5 mass% or less, further more preferably 3 mass% or less, and particularly preferably 2.5 mass% or less, based on the total mass of the granular gel capsules.

<14> The dermatological topical agent according to any one of <9> to <13>, wherein a content mass ratio of the component (a2) encapsulating agent in the granular gel capsules to the component (a1) UV absorber (preferably an oil-soluble UV absorber) in the granular gel capsules, [(a2)/(a1)], is preferably 0.001 or more, more preferably 0.003 or more, further more preferably 0.005 or more, and particularly preferably 0.01 or more, and is preferably 0.2 or less, more preferably 0.1 or less, further more preferably 0.08 or less, and particularly preferably 0.06 or less.

<15> The dermatological topical agent according to any one of <9> to <14>, wherein a content mass ratio of a total of all non-volatile oil agents in the granular gel capsules to the encapsulating agent in the component (a2) granular gel capsules, [(all non-volatile oil agents in granular gel capsules)/(a2)], is preferably 5 or more, more preferably 10 or more, further more preferably 15 or more, and particularly preferably 20 or more, and is preferably 105 or less, more preferably 95 or less, further more preferably 85 or less, and particularly preferably 75 or less.

<16> The dermatological topical agent according to any one of <3> to <15>, wherein the component (a4) is preferably one or more selected from the group consisting of ceramides, alcohols having from 12 to 22 carbon atoms, polyhydric alcohol mono C12-22 fatty acid esters, and polyhydric alcohol mono C12-22 alkyl ethers; more preferably one or more selected from the group consisting of alcohols having from 12 to 22 carbon atoms, polyhydric alcohol mono C12-22 fatty acid esters, and polyhydric alcohol mono C12-22 alkyl ethers; further more preferably one or more selected from the group consisting of monovalent alcohols having from 14 to 22 carbon atoms, glycerin mono C14-22 fatty acid esters, sorbitan mono C14-22 fatty acid esters, and mono C14-22 alkyl glyceryl ethers; further more preferably one or more selected from the group consisting of monovalent alcohols having from 16 to 22 carbon atoms, glycerin mono C16-22 fatty acid esters, sorbitan mono C16-22 fatty acid esters, and mono C16-22 alkyl glyceryl ethers; further more preferably one or more selected from the group consisting of monovalent alcohols having from 16 to 22 carbon atoms and glycerin mono C16-22 fatty acid esters; and particularly preferably one or more selected from the group consisting of cetyl alcohol, stearyl alcohol, and lipophilic glycerin monostearate.

<17> The dermatological topical agent according to any one of <3> to <16>, wherein a content of the component (a4) is preferably 0.1 mass% or more, more preferably 0.3 mass% or more, further more preferably 1 mass% or more, and particularly preferably 1.25 mass% or more, based on the total mass of the dermatological topical agent, and is preferably 12 mass% or less, more preferably 9 mass% or less, further more preferably 6 mass% or less, and particularly preferably 3 mass% or less, based on the total mass of the dermatological topical agent.

<18> The dermatological topical agent according to any one of <3> to <17>, wherein a content ratio of the component (a4) is preferably 0.1 mass% or more, more preferably 0.5 mass% or more, further more preferably 1 mass% or more, and particularly preferably 2 mass% or more, based on the total mass of the granular gel capsules, and is preferably 12.5 mass% or less, more preferably 10 mass% or less, further more preferably 7.5 mass% or less, and particularly preferably 5 mass% or less, based on the total mass of the granular gel capsules.

<19> The dermatological topical agent according to any one of <9> to <18>, wherein the component (a5) is preferably one or more selected from the group consisting of an aqueous thickener and an oily thickener.

<20> The dermatological topical agent according to <19>, wherein the aqueous thickener is preferably one or more selected from the group consisting of dextrin, methylcellulose, ethylcellulose, hydroxyethylcellulose, hydroxypropylcellulose, polyvinyl alcohol, polyacrylic acid, polymethacrylic acid, carboxyvinyl polymers, acrylic acid/alkyl acrylate copolymers, xanthan gum, carboxymethyl chitin, and chitosan; more preferably one or more selected from the group consisting of polyvinyl alcohol and (meth)acrylic acid-based polymers; further more preferably one or more selected from the group consisting of polyvinyl alcohol, carboxyvinyl polymers, and acrylic acid/alkyl acrylate copolymers; and particularly preferably one or more selected from the group consisting of polyvinyl alcohol and (acrylic acid/C10-30 alkyl acrylate) copolymers.

<21> The dermatological topical agent according to any one of <3> to <20>, wherein the oily thickener is preferably one or more selected from the group consisting of sugar fatty acid ester-based oily thickeners, polyglyceryl isostearate, glyceryl (behenate/eicosanedioate), and organically modified clay minerals; more preferably a sugar fatty acid ester-based oily thickener; further more preferably a dextrin fatty acid ester; and particularly preferably one or more selected from the group consisting of dextrin myristate, dextrin palmitate, dextrin stearate, dextrin (palmitate/2-ethylhexanoate), and dextrin (palmitate/hexyldecanoate).

<22> The dermatological topical agent according to any one of <3> to <21>, wherein a content of the oily thickener in the granular gel capsules is preferably 0.05 mass% or more, more preferably 0.1 mass% or more, further more preferably 0.3 mass% or more, and particularly preferably 0.5 mass% or more, based on the total mass of the dermatological topical agent, and is preferably 12 mass% or less, more preferably 9 mass% or less, further more preferably 6 mass% or less, and particularly preferably 2 mass% or less, based on the total mass of the dermatological topical agent.

<23> The dermatological topical agent according to any one of <3> to <22>, wherein a content ratio of the oily thickener in the granular gel capsules is preferably 0.1 mass% or more, more preferably 0.5 mass% or more, further more preferably 1 mass% or more, and particularly preferably 1.5 mass% or more, based on the total mass of the granular gel capsules, and is preferably 10 mass% or less, more preferably 7.5 mass% or less, further more preferably 5 mass% or less, and particularly preferably 3.5 mass% or less, based on the total mass of the granular gel capsules.

<24> The dermatological topical agent according to any one of <19> to <23>, wherein a content ratio of the aqueous thickener (including polyvinyl alcohol) in the entire dermatological topical agent is preferably 0.6 mass% or less, more preferably 0.55 mass% or less, further more preferably 0.5 mass% or less, and even more preferably 0.3 mass% or less, based on the total mass of the dermatological topical agent, and a content of the aqueous thickener other than polyvinyl alcohol in the entire dermatological topical agent is preferably 0.3 mass% or less, more preferably 0.25 mass% or less, further more preferably 0.13 mass% or less, even more preferably 0.06 mass% or less, and even more preferably 0.03 mass% or less, based on the total mass of the dermatological topical agent.

<25> The dermatological topical agent according to any one of <9> to <24>, wherein the component (a6) is preferably one or more selected from the group consisting of glycols and glycerins; more preferably glycerins; further more preferably one or more selected from the group consisting of glycerin, diglycerin, and polyglycerin; and particularly preferably glycerin.

<26> The dermatological topical agent according to any one of <9> to <25>, wherein a content ratio of the powder (a7) in the granular gel capsules is preferably 0 mass% or more and 40 mass% or less, more preferably 0 mass% or more and 30 mass% or less, further more preferably 0 mass% or more and 20 mass% or less, and particularly preferably 0 mass% or more and 10 mass% or less, based on the total mass of the granular gel capsules.

<27> The dermatological topical agent according to any one of <1> to <26>, wherein the granular gel capsules have an average particle size of preferably 20 μm or more, more preferably 30 μm or more, further more preferably 40 μm or more, further more preferably 60 μm or more, and particularly preferably 80 μm or more, and preferably 350 μm or less, more preferably 300 μm or less, further more preferably 250 μm or less, and particularly preferably 200 μm or less.

<28> The dermatological topical agent according to any one of <1> to <27>, wherein a content of the granular gel

capsules is preferably 15 mass% or more, more preferably 20 mass% or more, further more preferably 22.5 mass% or more, further more preferably 27.5 mass% or more, further more preferably 30 mass% or more, further more preferably 35 mass% or more, and particularly preferably 40 mass% or more, based on the total mass of the dermatological topical agent, and is preferably 80 mass% or less, more preferably 75 mass% or less, further more preferably 72.5 mass% or less, and particularly preferably 70 mass% or less, based on the total mass of the dermatological topical agent.

<29> The dermatological topical agent according to any one of <1> to <28>, wherein the component (b1) is preferably one or more selected from the group consisting of water and lower alcohols, and more preferably one or more selected from the group consisting of water, ethanol, and isopropanol.

<30> The dermatological topical agent according to any one of <1> to <29>, wherein the liquid preferably further comprises (b2) a cationic polymer.

<31> The dermatological topical agent according to <30>, wherein the component (b2) is preferably a silicone-based cationic polymer, more preferably one or more selected from the group consisting of poly(N-acylalkyleneimine)-modified silicone and amino-modified silicone, further more preferably poly(N-acylalkyleneimine)-modified silicone, and particularly preferably oxazoline-modified silicone.

<32> The dermatological topical agent according to <30> or <31>, wherein a content of the component (b2) is preferably 0 mass% or more, more preferably 0.005 mass% or more, further more preferably 0.01 mass% or more, further more preferably 0.02 mass% or more, and particularly preferably 0.05 mass% or more, based on the total mass of the dermatological topical agent, and is preferably 1 mass% or less, more preferably 0.6 mass% or less, further more preferably 0.3 mass% or less, and particularly preferably 0.1 mass% or less, based on the total mass of the dermatological topical agent.

<33> The dermatological topical agent according to any one of <1> to <32>, wherein a content mass ratio of the component (A) granular gel capsules to the component (B) liquid, [(A)/(B)], is preferably 0.1 or more, more preferably 0.2 or more, further more preferably 0.4 or more, and particularly preferably 0.5 or more, and is preferably 3.5 or less, more preferably 3 or less, further more preferably 2.5 or less, and particularly preferably 2 or less.

<34> The dermatological topical agent according to any one of <1> to <33>, wherein the content of the non-volatile oil agent in the liquid is preferably 0 mass% or more and 10 mass% or less, more preferably 0 mass% or more and 5 mass% or less, further more preferably 0 mass% or more and 1 mass% or less, and particularly preferably 0 mass%, based on the total mass of the dermatological topical agent.

<35> The dermatological topical agent according to any one of <1> to <34>, wherein a content mass ratio of all non-volatile oil agents in the liquid to a total of all non-volatile oil agents in the granular gel capsules, [(all non-volatile oil agent in liquid)/(all non-volatile oil agents in granular gel capsules)], is preferably 0 or more and 0.1 or less, more preferably 0 or more and 0.06 or less, further more preferably 0 or more and 0.01 or less, and particularly preferably 0.

<36> The dermatological topical agent according to any one of <1> to <35>, wherein the content of the aqueous thickener in the liquid is preferably 0 mass% or more and 0.03 mass% or less, more preferably 0 mass% or more and 0.015 mass% or less, further more preferably 0 mass% or more and 0.005 mass% or less, and particularly preferably 0 mass%, based on the total mass of the dermatological topical agent.

<37> The dermatological topical agent according to any one of <1> to <36>, wherein the content of the oily thickener in the liquid is preferably 0 mass% or more and 0.03 mass% or less, more preferably 0 mass% or more and 0.015 mass% or less, further more preferably 0 mass% or more and 0.005 mass% or less, and particularly preferably 0 mass%, based on the total mass of the dermatological topical agent.

<38> The dermatological topical agent according to any one of <1> to <37>, wherein a content of an emulsifier in the liquid is preferably 0 mass% or more and 0.4 mass% or less, more preferably 0 mass% or more and 0.2 mass% or less, further more preferably 0 mass% or more and 0.1 mass% or less, and particularly preferably 0 mass%, based on the total mass of the dermatological topical agent.

<39> The dermatological topical agent according to any one of <1> to <38>, wherein a total content of the non-volatile oil agent, aqueous thickener, and emulsifier in the liquid is preferably 0 mass% or more and 12 mass% or less, more preferably 0 mass% or more and 5 mass% or less, further more preferably 0 mass% or more and 0.001 mass% or less, and particularly preferably 0 mass%, based on the total mass of the dermatological topical agent.

<40> The dermatological topical agent according to any one of <1> to <39>, further comprising (C) a powder outside the granular gel capsules.

<41> The dermatological topical agent according to <40>, wherein the component (C) is preferably a powder other than UV scattering agents, more preferably one or more selected from the group consisting of cellulose and silica, further more preferably silica, and particularly preferably porous silica.

<42> The dermatological topical agent according to <40> or <41>, wherein a content of the component (C) is preferably 0.05 mass% or more, more preferably 0.2 mass% or more, further more preferably 0.6 mass% or more, and particularly preferably 1 mass% or more, based on the total mass of the dermatological topical agent, and is preferably 15 mass% or less, more preferably 12 mass% or less, further more preferably 9 mass% or less, and

particularly preferably 6 mass% or less, based on the total mass of the dermatological topical agent.

<43> The dermatological topical agent according to any one of <1> to <42>, which has a viscosity at 25°C of preferably 10 mPa·s or more, more preferably 20 mPa·s or more, further more preferably 25 mPa·s or more, and particularly preferably 30 mPa·s or more, and preferably 10 000 mPa·s or less, more preferably 5 000 mPa·s or less, further more preferably 1 000 mPa·s or less, and particularly preferably 500 mPa·s or less.

<44> The dermatological topical agent according to any one of <1> to <43>, which has a pH at 25°C of preferably 4 or more and 10 or less, more preferably 4.5 or more and 9 or less, and particularly preferably 5 or more and 8 or less.

<45> The dermatological topical agent according to any one of <1> to <44>, wherein the liquid is an aqueous composition.

<46> The dermatological topical agent according to any one of <1> to <45>, which is preferably a cosmetic, and more preferably a sunscreen.

## Examples

The present invention will be described in detail below with reference to Examples; however, the present invention is not limited to these Examples. In the Examples, various measurements and evaluations were performed as described below.

- Measurement Method and Evaluation Method

(1) Average Particle Size of Granular Gel Capsules

Using a laser scattering particle size distribution analyzer LA-960 (manufactured by HORIBA), the volume-based median diameter of each granular gel capsule was measured with a refractive index of 1.20.

(2) Viscosity

Each dermatological topical agent was placed in a glass bottle with a lid, and the viscosity at 25°C was measured the day after preparation. A B-type viscometer (TVB-10M), manufactured by Toki Sangyo Co., Ltd., was used in the measurement. After stirring was conducted in the glass bottle in advance, the viscosity was measured under the following conditions: rotor: No. 2, rotation speed: 30 rpm, measurement time: 1 minute. When the measurement could not be performed under these conditions, the rotor was changed to No. 4, then the measurement was performed.

(3) Fresh Feeling at Beginning of Application

Each dermatological topical agent was evaluated for whether it had light and fresh feeling at the beginning of application (i.e., whether it spread smoothly like water when spread on the skin or whether it felt like water when applied). Specifically, three expert panelists were asked to apply the dermatological topical agent to the inner side of the forearm, and to perform sensory evaluation on a 10-point scale, with 10 being "very fresh feeling at the beginning of application" and 1 being "no fresh feeling at all at the beginning of application". An average value of the obtained scores was determined.

(4) UV Protection Efficacy (UVPE)

Each dermatological topical agent was uniformly applied (3 cm × 3 cm) to the inner side of the forearm at 2 mg/cm$^2$ and dried in a cool and dark place for 15 minutes. After completion of drying, UV protection efficacy (UVPE) was derived using the multispectral UV polarization reflectance imaging system (MUPRIS) by the method described in the document (Nishino et al., Skin Research and Technology, 2019, Volume 25, Issue 5, pp. 639-652), and evaluated in accordance with the following criteria.

(Evaluation criteria of UV protection efficacy)

AA: a UVPE of 60 or more
A: a UVPE of 50 or more and less than 60
B: a UVPE of 40 or more and less than 50
C: a UVPE of less than 40

(Preparation Example 1: Granular Gel Capsules C1)

Granular gel capsules C1 were prepared to have the composition shown in Table 1, and the average particle size was measured. Table 1 shows the results.

The granular gel capsules were prepared in the following manner. Specifically, agar, an (acrylic acid/C10-30 alkyl acrylate) copolymer, polyvinyl alcohol, sodium hydroxide, and purified water were mixed and heated to 90°C to prepare a mixture I. Next, 2-ethylhexyl paramethoxycinnamate, hexyl diethylaminohydroxybenzoylbenzoate, bisethylhexyloxyphenol methoxyphenyl triazine, ethylhexyl triazone, dextrin palmitate, and glyceryl stearate were mixed by heating at 80°C to dissolve them to prepare a solution II. Then, the mixture I and the solution II were mixed and the mixture was stirred with a homomixer to prepare an oil-in-water type dispersion. The temperature of the obtained oil-in-water type dispersion was kept at 80°C, the oil-in-water type dispersion was sprayed into a gas phase at 25°C from a two-fluid spray nozzle (SUE45B, manufactured by Spraying Systems Co.), and allowed to stand until droplets of the oil-in-water type dispersion were cooled and solidified, thereby obtaining granular gel capsules.

(Preparation Examples 2 to 12: Granular Gel Capsules C2 to C12)

Granular gel capsules C2 to C12 with the compositions shown in Tables 1 and 2 were prepared in accordance with Preparation Example 1, and the average particle size was measured. Tables 1 and 2 show the results.

[Table 1]

| Component (mass%) | | Granular hydrogel capsules | | | | | |
|---|---|---|---|---|---|---|---|
| | | C1 | C2 | C3 | C4 | C5 | C6 |
| (a2) | Agar | 1.500 | 1.500 | 1.500 | 1.500 | 0.400 | 1.000 |
| (a5) | (Acrylic acid/C10-30 alkyl acrylate) copolymer *1 | 0.100 | 0.100 | 0.100 | 0.100 | 0.120 | 0.100 |
| | Polyvinyl alcohol *2 | 0.500 | 0.500 | 0.500 | 0.500 | 0.600 | 0.500 |
| | Sodium hydroxide | 0.030 | 0.030 | 0.030 | 0.030 | 0.030 | 0.030 |
| (a3) | Purified water | 64.270 | 64.270 | 64.270 | 54.103 | 65.250 | 64.770 |
| | Glycerin (86% Glycerin V (manufactured by Kao Corporation); active component: 86 mass%) | | | | | | |
| (a1) | 2-Ethylhexyl paramethoxycinnamate *3 | 17.000 | 17.000 | 17.000 | 17.000 | 17.000 | 17.000 |
| | Hexyl diethylaminohydroxybenzoylbenzoate *4 | 4.000 | 4.000 | 4.000 | 4.000 | 4.000 | 4.000 |
| | Bisethylhexyloxyphenol methoxyphenyl triazine *5 | 2.000 | 2.000 | 2.000 | 2.000 | 2.000 | 2.000 |
| | Ethylhexyl triazone *6 | 6.000 | 6.000 | 6.000 | 6.000 | 6.000 | 6.000 |
| (a5) | Dextrin palmitate *7 | 1.600 | 1.600 | 1.600 | 1.600 | 1600 | 1.600 |
| (a4) | Glyceryl stearate *8 | 3.000 | 3.000 | 3.000 | 3.000 | 3.000 | 3.000 |
| (a7) | Stearic acid-treated titanium oxide fine particles *9 | | | | 10.000 | | |
| | Polyhydroxy stearic acid *10 | | | | 0.167 | | |
| Total (mass%) | | 100 | 100 | 100 | 100 | 100 | 100 |
| Component (a1) (mass%) | | 29.000 | 29.000 | 29.000 | 29.000 | 29.000 | 29.000 |
| Component (a5) (mass%) | | 2.200 | 2.200 | 2.200 | 2.200 | 2.320 | 2.200 |
| Content mass ratio [(a2)/(a1)] | | 0.052 | 0.052 | 0.052 | 0.052 | 0.014 | 0.034 |
| Non-volatile oil agent in granular gel capsules (mass%) | | 33.600 | 33.600 | 33.600 | 33.600 | 33.600 | 33.600 |

(continued)

| Component (mass%) | Granular hydrogel capsules | | | | | |
|---|---|---|---|---|---|---|
| | C1 | C2 | C3 | C4 | C5 | C6 |
| Content mass ratio [(all non-volatile oil agents in granular gel capsules)/(a2)] | 22.400 | 22.400 | 22.400 | 22.400 | 84.000 | 33.600 |
| Content mass ratio ((a3)/(a1)\| | 2.216 | 2.216 | 2.216 | 1.866 | 2.250 | 2.233 |
| Content mass ratio [(a4)/(a1)] | 0.103 | 0.103 | 0.103 | 0.103 | 0.103 | 0.103 |
| Content mass ratio [(a5)/(a1)] | 0.076 | 0.076 | 0.076 | 0.076 | 0.080 | 0.076 |
| Average particle size (μm) | 100 | 40 | 250 | 100 | 100 | 100 |

[Table 2]

| Component (mass%) | | Granular hydrogel capsules | | | | | |
|---|---|---|---|---|---|---|---|
| | | C7 | C8 | C9 | C10 | C11 | C12 |
| (a2) | Agar | 3.000 | 1.500 | 1.500 | 1.500 | 1.500 | 0.400 |
| (a5) | (Acrylic acid/C10-30 alkyl acrylate) copolymer *1 | 0.100 | 0.066 | 0.140 | 0.066 | 0.120 | 0.120 |
| | Polyvinyl alcohol*2 | 0.500 | 0.328 | 0.730 | 0.328 | 0.600 | 0.600 |
| | Sodium hydroxide | 0.030 | 0.010 | 0.030 | 0.030 | 0.030 | 0.030 |
| (a3) | Purified water | 62.770 | 72.607 | 49.000 | 72.587 | 49.950 | 42.950 |
| | Glycerin (86% Glycerin V (manufactured by Kao Corporation); active component: 86 mass%) | | | | | 14.200 | 14.200 |
| (a1) | 2-Ethylhexyl paramethoxycinnamate *3 | 17.000 | 12.897 | 24.600 | 12.897 | 17.000 | 21.100 |
| | Hexyl diethylaminohydroxybenzoylbenzoate *4 | 4.000 | 3.034 | 5.800 | 3.034 | 4.000 | 5.000 |
| | Bisethylhexyloxyphenol methoxyphenyl triazine *5 | 2.000 | 1.517 | 2.900 | 1.517 | 2.000 | 2.500 |
| | Ethylhexyl triazone *6 | 6.000 | 4.552 | 8.700 | 4.552 | 6.000 | 7.400 |
| (a5) | Dextrin palmitate *7 | 1.600 | 1.214 | 2.300 | 1.214 | 1.600 | 2.000 |
| (a4) | Glyceryl stearate *8 | 3.000 | 2.276 | 4.300 | 2.276 | 3.000 | 3.700 |
| (a7) | Stearic acid-treated titanium oxide fine particles *9 | | | | | | |
| | Polyhydroxy stearic acid *10 | | | | | | |
| Total (mass%) | | 100 | 100 | 100 | 100 | 100 | 100 |
| Component (a1) (mass%) | | 29.000 | 22.000 | 42.000 | 22.000 | 29.000 | 36.000 |
| Component (a5) (mass%) | | 2.200 | 1.607 | 3.170 | 1.607 | 2.320 | 2.720 |
| Content mass ratio [(a2)/(a1)] | | 0.103 | 0.068 | 0.036 | 0.068 | 0.052 | 0.011 |
| Non-volatile oil agent in granular gel capsules (mass%) | | 33.600 | 25.490 | 48.600 | 25.490 | 33.600 | 41.700 |
| Content mass ratio [(all non-volatile oil agents in granular gel capsules)/(a2)] | | 11.200 | 16.993 | 32.400 | 16.993 | 22.400 | 104.250 |

(continued)

| Component (mass%) | Granular hydrogel capsules | | | | | |
|---|---|---|---|---|---|---|
| | C7 | C8 | C9 | C10 | C11 | C12 |
| Content mass ratio [(a3)/(a1)] | 2.164 | 3.300 | 1.167 | 3.299 | 1.722 | 1.193 |
| Content mass ratio [(a4)/(a1)] | 0.103 | 0.103 | 0.102 | 0.103 | 0.103 | 0.103 |
| Content mass ratio [(a5)/(a1)] | 0.076 | 0.073 | 0.075 | 0.073 | 0.080 | 0.076 |
| Average particle size (μm) | 100 | 100 | 100 | 100 | 100 | 100 |

(Examples 1 to 19 and Comparative Examples 1 to 3: Dermatological Topical Agents)

The dermatological topical agents of Examples 1 to 19 and Comparative Examples 1 to 3 were produced by a conventional method in accordance with the formulations shown in Tables 3 and 4, and the viscosity, fresh feeling at the beginning of application, and UV protection efficacy (UVPE) were measured and evaluated. Tables 3 and 4 show the results.

[Table 3]

| Component (mass%) | | | Example | | | | | | Example | | | | | |
|---|---|---|---|---|---|---|---|---|---|---|---|---|---|---|
| | | | 1 | 2 | 3 | 4 | 5 | 6 | 7 | 8 | 9 | 10 | 11 | 12 |
| (A) | Granular gel capsules | Capsule type | C1 | C2 | C3 | C4 | C5 | C6 | C7 | C8 | C9 | C10 | C1 | C11 |
| | | Content (mass%) | 50.000 | 50.000 | 50.000 | 50.000 | 50.000 | 50.000 | 50.000 | 65.900 | 34.530 | 50.000 | 38.000 | 50.000 |
| (b1) | Water | | 37.000 | 37.000 | 37.000 | 37.000 | 37.000 | 37.000 | 37.000 | 21.100 | 52.470 | 37.000 | 49.000 | 37.000 |
| (C) | Porous silica *11 | | 3.000 | 3.000 | 3.000 | 3.000 | 3.000 | 3.000 | 3.000 | 3.000 | 3.000 | 3.000 | 3.000 | 3.000 |
| (b1) | Ethanol | | 10.000 | 10.000 | 10.000 | 10.000 | 10.000 | 10.000 | 10.000 | 10.000 | 10.000 | 10.000 | 10.000 | 10.000 |
| | KOH | | | | | | | | | | | | | |
| (b2) | Poly(N-propionylethyleneimine)-modified silicone *12 | | | | | | | | | | | | | |
| Aqueous thickener | (Acrylic acid/C10-30 alkyl acrylate) copolymer *13 | | | | | | | | | | | | | |
| | (Acrylic acid/C10-30 alkyl acrylate) copolymer *1 | | | | | | | | | | | | | |
| Emulsifier | Polyoxyethylene (20) 2-hexyldecyl ether *14 | | | | | | | | | | | | | |
| UV absorber | 2-Ethylhexyl paramethoxycinnamate *3 | | | | | | | | | | | | | |
| | Hexyl diethylaminohydroxybenzoylbenzoate *4 | | | | | | | | | | | | | |
| | Bisethylhexyloxyphenol methoxyphenyl triazine *5 | | | | | | | | | | | | | |
| | Ethylhexyl triazone *6 | | | | | | | | | | | | | |
| Oily thickener | Dextrin palmitate *7 | | | | | | | | | | | | | |
| Amphiphilic solid fat | Glyceryl stearate *8 | | | | | | | | | | | | | |
| | Hydrogenated polyisobutene *15 | | | | | | | | | | | | | |
| Total (mass%) | | | 100 | 100 | 100 | 100 | 100 | 100 | 100 | 100 | 100 | 100 | 100 | 100 |
| Content of component (a1) (mass%) | | | 14.50 | 14.50 | 14.50 | 14.50 | 14.50 | 14.50 | 14.50 | 14.50 | 14.50 | 11.00 | 11.02 | 14.50 |

(continued)

| Component (mass%) | | Example | | | | | | Example | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| Content mass ratio [(a1)/(all UV absorbers)] | | 1.00 | 1.00 | 1.00 | 1.00 | 1.00 | 1.00 | 1.00 | 1.00 | 1.00 | 1.00 | 1.00 | 1.00 |
| Content of component (a2) (mass%) | | 0.75 | 0.75 | 0.75 | 0.75 | 0.20 | 0.50 | 1.50 | 0.99 | 0.52 | 0.75 | 0.57 | 0.75 |
| Content of component (B) (mass%) | | 47.00 | 47.00 | 47.00 | 47.00 | 47.00 | 47.00 | 47.00 | 31.10 | 62.47 | 47.00 | 59.00 | 47.00 |
| Content mass ratio [(A)/(B)] | | 1.06 | 1.06 | 1.06 | 1.06 | 1.06 | 1.06 | 1.06 | 2.12 | 0.55 | 1.06 | 0.64 | 1.06 |
| Content of non-volatile oil agent in granular gel capsules (mass%) | | 16.80 | 16.80 | 16.80 | 16.80 | 16.80 | 16.80 | 16.80 | 16.80 | 16.78 | 12.74 | 12.77 | 16.80 |
| Content of non-volatile oil agent in liquid (mass%) | | 0.00 | 0.00 | 0.00 | 0.00 | 0.00 | 0.00 | 0.00 | 0.00 | 0.00 | 0.00 | 0.00 | 0.00 |
| Content of aqueous thickener in liquid (mass%) | | 0.00 | 0.00 | 0.00 | 0.00 | 0.00 | 0.00 | 0.00 | 0.00 | 0.00 | 0.00 | 0.00 | 0.00 |
| UV protection efficacy (UVPE) | Measured value | 57 | 55 | 56 | 63 | 59 | 55 | 45 | 58 | 56 | 42 | 41 | 59 |
| | Evaluation | A | A | A | AA | A | A | B | A | A | B | B | A |
| Viscosity (mPa·s) | | 72 | 326 | 56 | 159 | 131 | 236 | 69 | 792 | 40 | 115 | 88 | 71 |
| Use impression (fresh feeling at beginning of application) | | 10.0 | 9.7 | 10.0 | 10.0 | 10.0 | 10.0 | 10.0 | 8.3 | 10.0 | 10.0 | 10.0 | 10.0 |
| pH at 25°C | | 6.9 | 7.1 | 7.1 | 6.8 | 6.8 | 6.8 | 6.8 | 6.8 | 6.3 | 6.8 | 6.8 | 6.8 |

[Table 4]

| Component (mass%) | | | Example | | | | | Com parative Example | | | | |
|---|---|---|---|---|---|---|---|---|---|---|---|---|
| | | | 13 | 14 | 15 | 16 | 17 | 18 | 19 | 1 | 2 | 3 |
| (A) | Granular gel capsules | Capsule type | C12 | C1 | C1 | C1 | C1 | C1 | C1 | C1 | C1 | - |
| | | Content (mass%) | 40.270 | 50.000 | 50.000 | 50.000 | 32.500 | 50.000 | 50.000 | 50.000 | 50.000 | |
| (b1) | Water | | 46.730 | 36.925 | 29.180 | 25.430 | 46.790 | 36.480 | 36.744 | 21.680 | 36.510 | 74.310 |
| (C) | Porous silica *11 | | 3.000 | 3.000 | 3.000 | 3.000 | 3.000 | 3.000 | 3.000 | 3.000 | 3.000 | |
| (b1) | Ethanol | | 10.000 | 10.000 | 10.000 | 10.000 | 10.000 | 10.000 | 10.000 | 10.000 | 10.000 | 10.000 |
| | KOH | | | | 0.070 | 0.070 | 0.070 | 0.070 | 0.056 | 0.070 | 0.110 | 0.070 |
| (b2) | Poly(N-propionylethyleneimine)-modified silicone *12 | | | 0.075 | | | | | | | | |
| Aqueous thickener | (Acrylic acid/C10-30 alkyl acrylate) copolymer *13 | | | | | | | | | | 0.380 | 0.120 |
| | (Acrylic acid/C10-30 alkyl acrylate) copolymer *1 | | | | 0.250 | 0.250 | 0.250 | 0.250 | 0.200 | 0.250 | | 0.100 |
| Emulsifier | Polyoxyethylene (20) 2-hexyldecyl ether *14 | | | | | | | 0.200 | | | | |
| UV absorber | 2-Ethylhexyl paramethoxycinnamate *3 | | | | | | 2.975 | | | | | 8.500 |
| | Hexyl diethylaminohydroxybenzoylbenzoate | | | | | | 0.700 | | | | | 2.000 |
| | Bisethylhexyloxyphenol methoxyphenyl triazine *5 | | | | | | 0.350 | | | | | 1.000 |
| | Ethylhexyl triazone *6 | | | | | | 1.050 | | | | | 3.000 |
| Oily thickener | Dextrin palmitate *7 | | | | | | 0.263 | | | | | 0.750 |
| Amphiphilic solid fat | Glyceryl stearate *8 | | | | | | 0.053 | | | | | 0.150 |
| | Hydrogenated polyisobutene *15 | | | | 7.500 | 11.250 | 2.000 | | | 15.000 | | |
| Total (mass%) | | | 100 | 100 | 100 | 100 | 100 | 100 | 100 | 100 | 100 | 100 |
| Content of component (a1) (mass%) | | | 14.50 | 14.50 | 14.50 | 14.50 | 9.43 | 14.50 | 14.50 | 14.50 | 14.50 | 0.00 |
| Content mass ratio [(a1)/(all UV absorbers)] | | | 1.00 | 1.00 | 1.00 | 1.00 | 0.65 | 1.00 | 1.00 | 1.00 | 1.00 | 0.00 |
| Content of component (a2) (mass%) | | | 0.16 | 0.75 | 0.75 | 0.75 | 0.49 | 0.75 | 0.75 | 0.75 | 0.75 | 0.00 |

(continued)

| Component (mass%) | | Example | | | | | Com parative Example | | | | |
|---|---|---|---|---|---|---|---|---|---|---|---|
| | | 13 | 14 | 15 | 16 | 17 | 18 | 19 | 1 | 2 | 3 |
| Content of component (B) (mass%) | | 56.73 | 47.00 | 47.00 | 47.00 | 64.50 | 47.00 | 47.00 | 47.00 | 47.00 | 100.00 |
| Content mass ratio [(A)/(B)] | | 0.71 | 1.06 | 1.06 | 1.06 | 0.50 | 1.06 | 1.06 | 1.06 | 1.06 | 0.00 |
| Content of non-volatile oil agent in granular gel capsules (mass%) | | 16.79 | 16.80 | 16.80 | 16.80 | 10.92 | 16.80 | 16.80 | 16.80 | 16.80 | 0.00 |
| Content of non-volatile oil agent in liquid (mass%) | | 0.00 | 0.00 | 7.50 | 11.25 | 7.39 | 0.00 | 0.00 | 15.00 | 0.00 | 15.40 |
| Content of aqueous thickener in liquid (mass%) | | 0.00 | 0.00 | 0.25 | 0.25 | 0.25 | 0.25 | 0.20 | 0.25 | 0.38 | 0.22 |
| UV protection efficacy (UVPE) | Measured value | 58 | 58 | 48 | 44 | 45 | 52 | 53 | 40 | 54 | 37 |
| | Evaluation | A | A | B | B | B | A | A | B | A | C |
| Viscosity (mPa·s) | | 525 | 331 | 2460 | 2980 | 2450 | 2540 | 2870 | 3180 | 9460 | 1500 |
| Use impression (fresh feeling at beginning of application) | | 8.3 | 9.3 | 7.0 | 6.7 | 6.7 | 7.0 | 7.3 | 3.7 | 1.0 | 7.0 |
| pH at 25°C | | 6.4 | 6.8 | 6.6 | 6.6 | 6.6 | 6.8 | 6.8 | 6.6 | 6.7 | 6.7 |

The symbols in the tables each indicate the following.

*1: PEMULEN TR-2 (manufactured by Lubrizol Advanced Materials, Inc.)
*2: GOHSENOL EG-05 (manufactured by Mitsubishi Chemical Corporation)
*3: UVINUL MC-80 (manufactured by BASF)
*4: UVINUL A PLUS GRANULAR (manufactured by BASF)
*5: TINOSORB S (manufactured by BASF)
*6: UVINUL T150 (manufactured by BASF)
*7: RHEOPEARL KL2 (manufactured by Chiba Flour Milling Co., Ltd.)
*8: MONTEX A (manufactured by Miyoshi Oil & Fat Co., Ltd.)
*9: MT-100Z (manufactured by Tayca Corporation)
*10: SALACOSHS-6C (manufactured by The Nisshin OilliO Group, Ltd.)
*11: SUNSPHERE H-121 (manufactured by AGC Si-Tech Co., Ltd.)
*12: a product prepared in accordance with the disclosure of Synthesis Example 1 in JP-A-2008-143820
*13: PEMULEN TR-1 (manufactured by Lubrizol Advanced Materials, Inc.)
*14: EMULGEN 1620G (manufactured by Kao Corporation)
*15: PARLEAM EX (manufactured by NOF Corporation)

(5) Redispersibility

The dermatological topical agents of Examples 1 and 14 were also evaluated for redispersibility.

Specifically, 50 mL of the dermatological topical agent of Example 1 or 14 was placed in a 50-mL glass bottle, and stainless steel stirring balls with a diameter of 5 mm were added to the glass bottle. Next, after being allowed to stand at room temperature for 72 hours, the number of times of shaking required until the stainless steel stirring balls were stirred to obtain a homogeneous state was counted. Table 5 shows the results. A smaller number of times of shaking is considered to indicate higher redispersibility.

[Table 5]

| | Example 1 | Example 14 |
|---|---|---|
| Redispersibility (number of times of shaking) | >10 | 1 |

(6) Storage Stability (Viscosity during Low-Temperature Storage)

The dermatological topical agents of Examples 1 and 2 were also evaluated for storage stability (viscosity) during low-temperature storage.

Specifically, the dermatological topical agent of Example 1 or 2 was stored at 5°C for 6 months, then returned to room temperature, and shaken until homogeneous. Thereafter, the storage stability was evaluated based on changes in viscosity before and after storage. The viscosity after storage was measured in the same manner as for the viscosity on the day after preparation, and the rate of increase (%) in viscosity after storage relative to viscosity before storage was calculated. Table 6 shows the results. A smaller rate of increase indicates higher storage stability.

[Table 6]

| | Example 1 | Example 2 |
|---|---|---|
| Storage stability | 138% | 190% |

(Examples 20 to 22: Dermatological Topical Agents)

The dermatological topical agents of Examples 20 to 22 were produced in the same manner as in Example 1, except that the contents of water, porous silica, and poly(N-propionylethyleneimine)-modified silicone were changed as shown in Table 7. Then, the viscosity was measured. Table 7 shows the results.

(7) Storage Stability (Appearance during High-Temperature Storage)

The dermatological topical agents of Examples 1, 14, and 20 to 22 were evaluated for storage stability during high-temperature storage.

Specifically, the dermatological topical agents shown in Table 7 were stored at 50°C for 1 month. The dermatological topical agents after storage were visually observed and evaluated in accordance with the following criteria. Table 7 shows the results.

(Evaluation criteria of stability (appearance) during high-temperature storage)

A: No oil separation was observed in the supernatant before shaking.
B: Oil separation was observed in the supernatant before shaking, but after shaking, the appearance was the same as before storage.

[Table 7]

| Component (mass%) | | | Example | | | | |
|---|---|---|---|---|---|---|---|
| | | | 1 | 20 | 14 | 21 | 22 |
| (A) | Granular gel capsules | Capsule type | C1 | C1 | C1 | C1 | C1 |
| | | Content (mass%) | 50.000 | 50.000 | 50.000 | 50.000 | 50.000 |
| (b1) | Water | | 37.000 | 36.700 | 36.925 | 36.970 | 39.970 |
| (C) | Porous silica *11 | | 3.000 | 3.000 | 3.000 | 3.000 | |
| (b1) | Ethanol | | 10.000 | 10.000 | 10.000 | 10.000 | 10.000 |
| | KOH | | | | | | |
| (b2) | Poly(N-propionylethyleneimine)-modified silicone *12 | | | 0.300 | 0.075 | 0.030 | 0.030 |
| Aqueous thickener | (Acrylic acid/C10-30 alkyl acrylate) copolymer *13 | | | | | | |
| | (Acrylic acid/C10-30 alkyl acrylate) copolymer *1 | | | | | | |
| Emulsifier | Polyoxyethylene (20) 2-hexyldecyl ether *14 | | | | | | |
| UV absorber | 2-Ethylhexyl paramethoxycinnamate *3 | | | | | | |
| | Hexyl diethylaminohydroxybenzoylbenzoate *4 | | | | | | |
| | Bisethylhexyloxyphenol methoxyphenyl triazine *5 | | | | | | |
| | Ethylhexyl triazone *6 | | | | | | |
| Oily thickener | Dextrin palmitate *7 | | | | | | |
| Amphiphilic solid fat | Glyceryl stearate *8 | | | | | | |
| | Hydrogenated polyisobutene *15 | | | | | | |
| Total (mass%) | | | 100 | 100 | 100 | 100 | 100 |
| Viscosity (mPa·s) | | | 72 | 338 | 331 | 152 | 101 |
| Appearance evaluation after storage at 50°C for 1 month | | | B | A | A | A | B |

(8) Adhesive Feeling in Later Stages of Application

After the dermatological topical agents of Examples 1 and 19, and Comparative Example 2 were each dropped

on the inner side of the upper arm at 2 mg/cm$^2$, a finger with an inertial sensor (described in JP-A-2018-109599) moved back and forth at a constant application speed (27 cm/sec), and the application was continued until a stop sensation (easiness to absorb) was felt, while obtaining a sensor value Gx at a sampling interval of 1 ms. After the obtained Gx was converted to absolute values, the maximum value was taken every 1 s, and the rate of change of the maximum value over time from the time when it was felt that easiness to absorb began to the time when it was felt that easiness to absorb ended was defined as the amount of change in texture ($\Delta G_x$).

Table 8 shows the results. A larger value of $\Delta G_x$ indicates a more significant increase in the angular velocity Gx in the twisting direction of the finger during application, and adhesive feeling in the later stages of application is felt. The change in texture makes the end of application more palpable. The shorter the time from the start of application to the end of easiness to absorb, the more quickly the dermatological topical agent was compatible with the skin.

[Table 8]

| | Example | Example | Comparative Example |
|---|---|---|---|
| | 1 | 19 | 2 |
| Time when easiness to absorb began (s) | 21 | 21 | 20 |
| Time when easiness to absorb ended (s) | 27 | 29 | 33 |
| Inertial sensor $\Delta G_X$ | 13.3 | 9.3 | 6.3 |

(Preparation Example 13: Granular Gel Capsules C13)

Granular gel capsules C13 with the composition shown in Table 9 were prepared in accordance with Preparation Example 1.

[Table 9]

| Component (mass%) | | Granular hydrogel capsules |
|---|---|---|
| | | C13 |
| (a2) | Agar | 1.500 |
| (a5) | (Na acrylate/Na acryloyldimethyltaurate) copolymer *16 | 0.100 |
| | Polyvinyl alcohol *2 | 0.500 |
| (a3) | Purified water | 64.300 |
| (a8) | Isopropyl palmitate *17 | 5.000 |
| | Hydrogenated polyisobutene *15 | 5.000 |
| | C12-15 Alkyl benzoate *18 | 6.000 |
| | Neopentyl glycol dicaprate *19 | 6.000 |
| | Isononyl isononanoate *20 | 5.000 |
| | Olive fruit oil *21 | 1.000 |
| | Vaseline *22 | 1.000 |
| (a5) | Dextrin palmitate *7 | 1.600 |
| (a4) | Glyceryl stearate *8 | 3.000 |
| Total (mass%) | | 100 |
| Component (a5) (mass%) | | 2.200 |
| Non-volatile oil agent in granular gel capsules (mass%) | | 33.600 |
| Content mass ratio [(all non-volatile oil agents in granular gel capsules)/(a2)] | | 22.400 |

The symbols in the table each indicate the following.

*2: GOHSENOL EG-05 (manufactured by Mitsubishi Chemical Corporation)
*7: RHEOPEARL KL2 (manufactured by Chiba Flour Milling Co., Ltd.)
*8: MONTEX A (manufactured by Miyoshi Oil & Fat Co., Ltd.)
*15: PARLEAM EX (manufactured by NOF Corporation)
*16: SIMULGEL EG QD (manufactured by SEPPIC)
*17: EXCEPARL IPP (manufactured by Kao Corporation)
*18: FINSOLV TN (manufactured by Innospec Performance Chemicals)
*19: ESTEMOL N-01 (manufactured by The Nisshin OilliO Group, Ltd.)
*20: SALACOS 99 (manufactured by The Nisshin OilliO Group, Ltd.)
*21: CROPURE OL-LQ-(JP) (manufactured by Croda International Plc)
*22: SUPER WHITE PROTOPET (Sonneborn, LLC)

(Example 23: Dermatological Topical Agent)

The dermatological topical agent of Example 23 was produced by a general method in accordance with the formulation shown in Table 10, and the fresh feeling at the beginning of application was evaluated. Table 10 shows the results.

[Table 10]

| Component (mass%) | | | Example 23 |
|---|---|---|---|
| (A) | Granular gel capsules | Capsule type | C13 |
| | | Content (mass%) | 50.000 |
| (b1) | Water | | 37.000 |
| (C) | Porous silica *11 | | 3.000 |
| (b1) | Ethanol | | 10.000 |
| Total (mass%) | | | 100 |
| Content of component (a1) (mass%) | | | 0.00 |
| Content mass ratio [(a1)/(all UV absorbers)] | | | - |
| Content of component (a2) (mass%) | | | 0.75 |
| Content of component (B) (mass%) | | | 47.00 |
| Content mass ratio [(A)/(B)] | | | 1.06 |
| Content of non-volatile oil agent in granular gel capsules (mass%) | | | 16.80 |
| Content of non-volatile oil agent in liquid (mass%) | | | 0.00 |
| Content of aqueous thickener in liquid (mass%) | | | 0.00 |
| Use impression (fresh feeling at beginning of application) | | | 5.7 |

(9) Uniformity of Coating Film

Among the components of the granular gel capsules C13, 0.01 mass% of water was replaced with 0.01 mass% of a fluorescent dye Nile Red (manufactured by Tokyo Chemical Industry Co., Ltd.), and granular gel capsules C13-1 containing a non-volatile oil agent stained with Nile Red were prepared. Among the components of the dermatological topical agent of Example 23, the granular gel capsules C13 were replaced with C13-1 to produce an agent α. The agent α was applied to a model plate (HELIOPLATE HD6, manufactured by Helioscreen) at 2 mg/$cm^2$.

The coating films of a model plate not coated with a dermatological topical agent and the model plate coated with the agent α were observed with a fluorescence microscope (BZ-X810, manufactured by Keyence Corporation).

Fig. 1 shows a micrograph of the dermatological topical agent-uncoated model plate, and Fig. 2 shows a fluorescence micrograph of the agent α-coated model plate (in Fig. 2, a higher color density indicates stronger coloring and a thicker coating film, and a lower color density indicates weaker coloring and a thinner coating film). In the fluorescence micrograph of the agent α-coated model plate, the color developed evenly, regardless of the unevenness of the surface of the model plate, and the difference in color density was small over the entire area. The results shown in Figs.

1 and 2 confirmed that the fluorescent non-volatile oil agent stained with Nile Red was evenly present on the recesses and protrusions of the surface of the model plate; that is, the uniformity of the coating film was confirmed.

## Claims

1. A dermatological topical agent comprising granular gel capsules comprising a non-volatile oil agent, and a liquid comprising the following component (bl):
(b1) a volatile medium,

   wherein the granular gel capsules are dispersed in the liquid,
   a content of the non-volatile oil agent in the granular gel capsules is 1.5 mass% or more based on a total mass of the dermatological topical agent,
   a content of the non-volatile oil agent in the liquid is 0 mass% or more and 14 mass% or less based on the total mass of the dermatological topical agent, and
   a content of an aqueous thickener in the liquid is 0 mass% or more and 0.25 mass% or less based on the total mass of the dermatological topical agent.

2. The dermatological topical agent according to claim 1, wherein the granular gel capsules comprise, as the non-volatile oil agent, one or more selected from the group consisting of (a1) an oil-soluble UV absorber, (a4) an amphiphilic solid fat, (a5-1) an oily thickener, and (a8) a non-volatile oil agent excluding oil-soluble UV absorbers, amphiphilic solid fats, and oily thickeners.

3. The dermatological topical agent according to claim 1, wherein the granular gel capsules comprise at least (a1) an oil-soluble UV absorber as the non-volatile oil agent.

4. The dermatological topical agent according to claim 3, wherein a content mass ratio of the component (a1) to a total of all UV absorbers in the dermatological topical agent, [(al)/(all UV absorbers)], is 0.85 or more and 1 or less.

5. The dermatological topical agent according to any one of claims 1 to 4, wherein the granular gel capsules further comprise (a2) an encapsulating agent.

6. The dermatological topical agent according to any one of claims 1 to 5, wherein the granular gel capsules comprise at least (a4) an amphiphilic solid fat as the non-volatile oil agent, and a content ratio of the component (a4) is 0.1 mass% or more based on a total mass of the granular gel capsules.

7. The dermatological topical agent according to any one of claims 1 to 6, wherein the granular gel capsules comprise at least (a5-1) an oily thickener as the non-volatile oil agent, and a content ratio of the component (a5-1) is 0.1 mass% or more based on the total mass of the granular gel capsules.

8. The dermatological topical agent according to any one of claims 1 to 7, wherein a content ratio of (a7) a powder in the granular gel capsules is 0 mass% or more and 30 mass% or less based on the total mass of the granular gel capsules.

9. The dermatological topical agent according to any one of claims 1 to 8, wherein the granular gel capsules have an average particle size of 60 μm or more and 350 μm or less.

10. The dermatological topical agent according to any one of claims 1 to 9, wherein a content of the granular gel capsules is 27.5 mass% or more based on the total mass of the dermatological topical agent.

11. The dermatological topical agent according to any one of claims 1 to 10, wherein the content of the aqueous thickener in the liquid is 0 mass% or more and 0.005 mass% or less based on the total mass of the dermatological topical agent, and a content of the oily thickener in the liquid is 0 mass% or more and 0.005 mass% or less based on the total mass of the dermatological topical agent.

12. The dermatological topical agent according to any one of claims 1 to 11, wherein a content of an emulsifier in the liquid is 0 mass% or more and 0.4 mass% or less based on the total mass of the dermatological topical agent.

**13.** The dermatological topical agent according to any one of claims 1 to 12, wherein the liquid further comprises (b2) a cationic polymer.

**14.** The dermatological topical agent according to any one of claims 1 to 13, further comprising a powder outside the granular gel capsules.

**15.** The dermatological topical agent according to claim 14, wherein the powder outside the granular gel capsules is silica.

**16.** The dermatological topical agent according to any one of claims 1 to 15, wherein a content mass ratio of all non-volatile oil agents in the liquid to a total of all non-volatile oil agents in the granular gel capsules, [(all non-volatile oil agents in liquid)/(all non-volatile oil agents in granular gel capsules)], is 0 or more and 0.1 or less.

**17.** The dermatological topical agent according to any one of claims 1 to 16, which has a viscosity at 25°C of 10 000 mPa·s or less.

Fig. 1

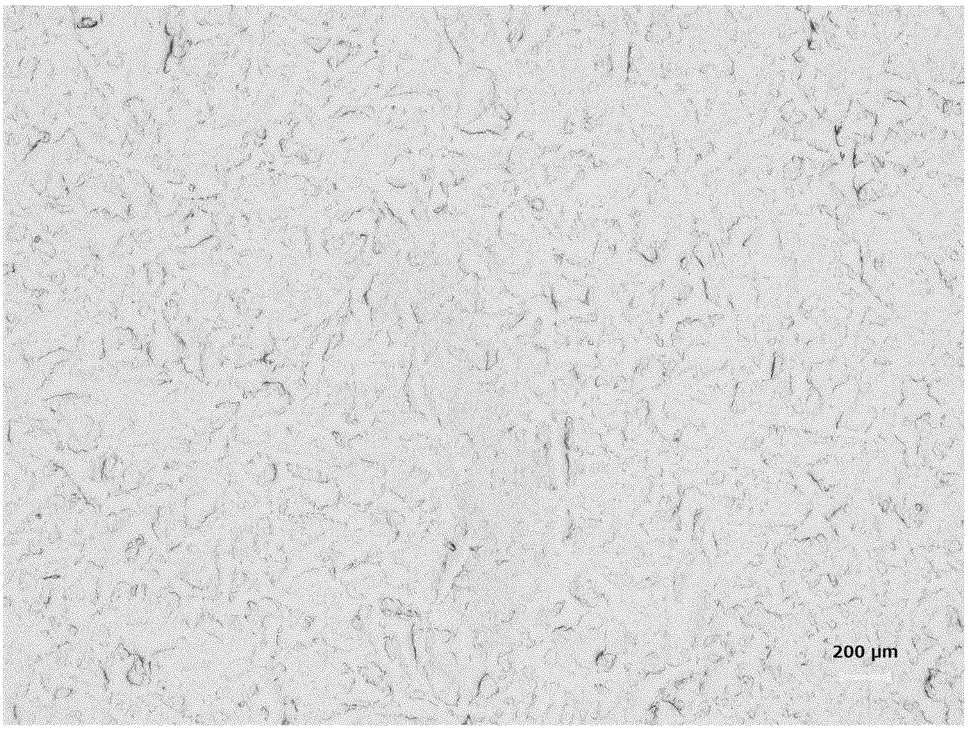


Fig. 2

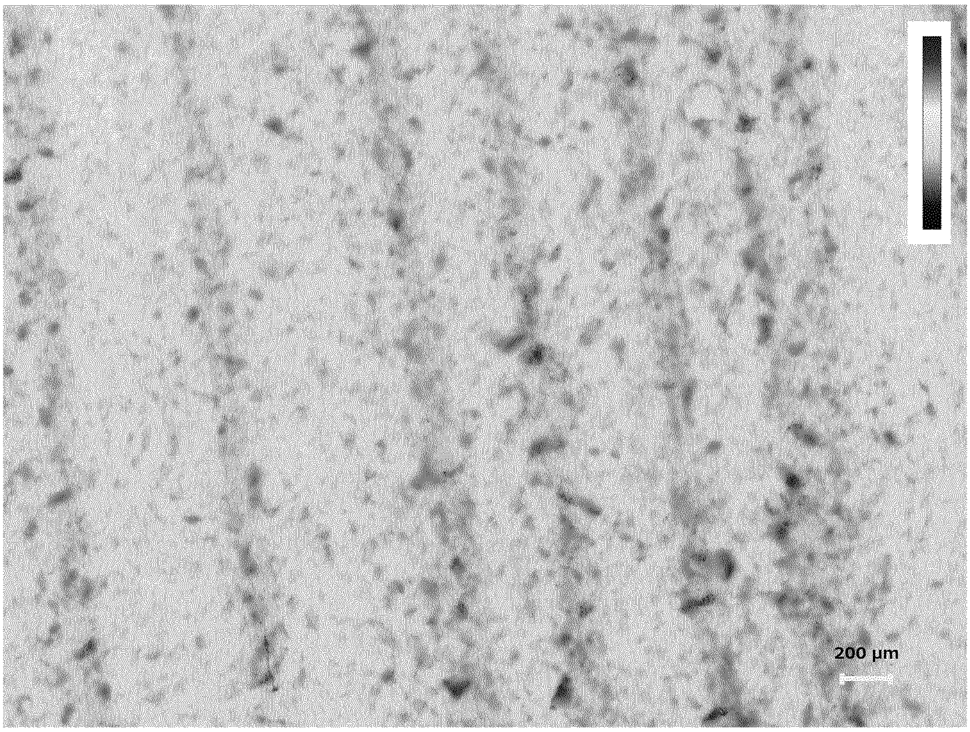

## INTERNATIONAL SEARCH REPORT

International application No.
**PCT/JP2022/022097**

**A. CLASSIFICATION OF SUBJECT MATTER**

***A61Q 17/04***(2006.01)i; ***A61K 8/11***(2006.01)i; ***A61K 8/25***(2006.01)i; ***A61K 8/34***(2006.01)i; ***A61K 8/37***(2006.01)i; ***A61K 8/73***(2006.01)i; ***A61K 8/81***(2006.01)i; ***A61K 8/898***(2006.01)i
FI: A61K8/11; A61K8/73; A61K8/81; A61Q17/04; A61K8/37; A61K8/34; A61K8/898; A61K8/25

According to International Patent Classification (IPC) or to both national classification and IPC

**B. FIELDS SEARCHED**

Minimum documentation searched (classification system followed by classification symbols)

A61Q17/04; A61K8/11; A61K8/25; A61K8/34; A61K8/37; A61K8/73; A61K8/81; A61K8/898

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2016-104712 A (KAO CORP) 09 June 2016 (2016-06-09) claims, paragraphs [0008], [0063], [0065], [0068], [0097], [0099] | 1-10, 12<br>11, 14-15, 17 |
| Y | JP 2003-500428 A (SOL-GEL TECHNOLOGIES LTD) 07 January 2003 (2003-01-07) claims, paragraphs [0081]-[0091], examples 6-9 | 11, 14-15, 17 |
| Y | JP 2012-171892 A (KAO CORP) 10 September 2012 (2012-09-10) paragraphs [0037]-[0039], [0046]-[0050] | 14-15 |
| A | JP 2011-148771 A (KAO CORP) 04 August 2011 (2011-08-04) entire text | 1-17 |
| A | JP 2001-096146 A (SHISEIDO CO LTD) 10 April 2001 (2001-04-10) entire text, all drawings | 1-17 |
| A | JP 2001-524123 A (KOBO PRODUCTS, INC.) 27 November 2001 (2001-11-27) entire text, all drawings | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 July 2022** | **16 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/022097**

**C. DOCUMENTS CONSIDERED TO BE RELEVANT**

| Category* | Citation of document, with indication, where appropriate, of the relevant passages | Relevant to claim No. |
|---|---|---|
| A | CN 108743496 A (PROYA COSMETICS CO LTD) 06 November 2018 (2018-11-06) entire text | 1-17 |

Form PCT/ISA/210 (second sheet) (January 2015)

**INTERNATIONAL SEARCH REPORT**
**Information on patent family members**

International application No.
**PCT/JP2022/022097**

| Patent document cited in search report | Publication date (day/month/year) | Patent family member(s) | Publication date (day/month/year) |
|---|---|---|---|
| JP 2016-104712 A | 09 June 2016 | (Family: none) | |
| JP 2003-500428 A | 07 January 2003 | US 2002/0037261 A1<br>claims, paragraphs [0097]-[0107], examples 6-9<br>WO 2000/0072806 A1<br>EP 1181001 A2 | |
| JP 2012-171892 A | 10 September 2012 | (Family: none) | |
| JP 2011-148771 A | 04 August 2011 | US 2012/0288457 A1<br>WO 2011/077674 A1<br>EP 2517689 A1<br>CN 102665652 A | |
| JP 2001-096146 A | 10 April 2001 | US 6391288 B1<br>EP 1072259 A2<br>CN 1287877 A<br>KR 10-2001-0049883 A | |
| JP 2001-524123 A | 27 November 2001 | US 2002/0086042 A1<br>WO 1998/050000 A2<br>EP 0979066 A2 | |
| CN 108743496 A | 06 November 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002037713 A
- JP 3193716 A
- JP 2012171891 A
- JP 2012171892 A
- JP 2014091737 A
- JP 2016104712 A
- JP 2212579 A
- JP 3220153 A
- JP 2013053146 A
- JP 2008143820 A
- JP 2009024114 A
- JP 2015067603 A
- JP 2016204336 A
- JP 2018109599 A


### Non-patent literature cited in the description


- *J. Lipid Res,* 1983, vol. 24, 759
- *J. Lipid. Res,* 1994, vol. 35, 2069
- **NISHINO et al.** *Skin Research and Technology,* 2019, vol. 25 (5), 639-652